(19)

Europäisches
Patentamt

European
Patent Office

Office européen
des brevets

(11) **EP 4 266 195 A1**

(12) **EUROPEAN PATENT APPLICATION**

(43) Date of publication:
    **25.10.2023 Bulletin 2023/43**

(21) Application number: **22168875.7**

(22) Date of filing: **19.04.2022**

(51) International Patent Classification (IPC):
    $G06F\ 16/45^{(2019.01)}$   $G06F\ 16/55^{(2019.01)}$
    $G16H\ 30/00^{(2018.01)}$   $G16H\ 50/20^{(2018.01)}$
    $G06N\ 3/04^{(2023.01)}$   $G06N\ 3/08^{(2023.01)}$

(52) Cooperative Patent Classification (CPC):
    **G06F 16/55; G06F 16/45; G06N 3/045; G06N 3/08;
    G16H 15/00; G16H 30/40; G16H 50/20;
    G16H 50/70**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **KH MA MD TN**

(71) Applicant: **Microsoft Technology Licensing, LLC
    Redmond, WA 98052-6399 (US)**

(72) Inventors:
    • **OKTAY, Ozan
      Redmond, 98052-6399 (US)**
    • **VALLE, Javier Alvarez
      Redmond, 98052-6399 (US)**
    • **USUYAMA, Naoto
      Redmond, 98052-6399 (US)**

    • **BANNUR, Shruthi Jaisimha
      Redmond, 98052-6399 (US)**
    • **POON, Hoifung
      Redmond, 98052-6399 (US)**
    • **NORI, Aditya
      Redmond, 98052-6399 (US)**
    • **SCHWAIGHOFER, Anton
      Redmond, 98052-6399 (US)**
    • **COELHO DE CASTRO, Daniel
      Redmond, 98052-6399 (US)**
    • **HYLAND, Stephanie
      Redmond, 98052-6399 (US)**
    • **NAUMANN, Tristan
      Redmond, 98052-6399 (US)**

(74) Representative: **Page White Farrer
    Bedford House
    21a John Street
    London WC1N 2BF (GB)**

(54) **TRAINING OF TEXT AND IMAGE MODELS**

(57)     A method of training a text model using a plurality of text passage combinations, each text passage combination comprising a respective first text passage and a respective second text passage describing a same matter as the respective first text passage but being differently worded than the respective first text passage. The training comprises minimizing a measure of statistical difference between a respective value of a first text embedding and the corresponding value of a second text embedding over the plurality of text passage combinations. The method then comprises jointly training the text model and an image model based on plurality of image-text combinations, each comprising a respective image and a respective textual report describing the respective image. The joint training comprises minimizing a measure of statistical difference between the value of an image embedding and the corresponding value of a third text embedding over the plurality of image-text combinations.

<u>Figure 2A</u>

## Description

### Background

A condition of a human or animal subject (e.g. patient) is often estimated at least in part based on one or more bodily scans, which produce images of the subject's body using imaging technologies such as x-ray, positron emission tomography (PET), magnetic resonance imaging (MRI), ultrasound or microscopy. For example such scans could be used to diagnose an illness of the subject, such as cancer.

Conventionally, a human practitioner (e.g. radiologist) reviews the one or more scans and then manually writes a report giving his or her assessment of the scans. This may include for example a possible diagnosis of one or more conditions which the scan may reveal, and a written explanation of which features in the scan are indicative of the possible diagnosis. For example a radiologist reviewing a chest x-ray might write "A mass is present in the left lower lobe and therefore malignancy must be considered".

More recently machine learning (ML) has been applied to try to improve the quality of such reports. Machine learning is a form of artificial intelligence (AI) which learns to produce a desired output based on training data, using either a supervised, reinforcement or unsupervised approach. For example a common form of ML model is a neural network, often employing a layered approach known as deep learning. Advances in machine learning have enabled automated diagnosis systems that may be used to assist medical professionals and thus improve healthcare workflows, such as to reduce errors and omissions.

In fields such as medicine multi-modal data is often available, e.g. radiology images and corresponding reports. Such data can be used to train a machine learning model to perform automated image analysis. Examples of such models include GLoRIA and ConVIRT. These use image and text encoders to learn to match text to images at a pretraining stage. The pretrained encoders are then fine-tuned with labels in a supervised approach. After training, in the deployment stage, the model can be used to perform image-text retrieval and classification. An object detector or segmentation module is also placed at the output of the image encoder in order to separately detect objects within the images being studied or to segment regions of the images.

Automated image analysis or vision-language processing may also be of interest in other fields, not just medicine, e.g. for performing (at least partially) automated analysis of physical objects, such as diagnostics of mechanical, electronic or electrical devices.

### Summary

It is recognized herein that there is still scope for the improvements in efficiency of vision-language processing compared to existing automated image analysis models such as GLoRIA and ConVIRT.

One way to improve efficiency would be to make the most of available text data and parse its valuable content to a greater extent to supervise the image model.

According to one aspect disclosed herein, there is provided computer-implemented method comprising: accessing a machine learning model comprising an image model and a text model; accessing a plurality of text passage combinations, each text passage combination comprising a respective first text passage and a respective second text passage describing a same matter as the respective first text passage but being differently worded than the respective first text passage; and performing first training of the text model, wherein the first training comprises: inputting each first text passage into the text model in order to generate a respective value of a first text embedding, inputting each second text passage into the text model in order to generate a corresponding value of a second text embedding, and training the text model to minimize a measure of statistical difference between the value of the first text embedding and the corresponding value of the second text embedding over the plurality of text passage combinations. The method further comprises accessing a plurality of image-text combinations, each comprising a respective image and a respective textual report describing the respective image; and after the first training, performing joint training of the image model and text model, the joint training comprising inputting each image into the image model in order to generate a respective value of an image embedding, inputting each textual report into the text model in order to generate a corresponding value of a third text embedding, and training the image model and text model to minimize a measure of statistical difference between the value of the image embedding and the corresponding value of the third text embedding over the plurality of image-text combinations.

The disclosed method thus employs a "pre-training" of the text model which exploits the existence of pre-existing text data, combined with contrastive learning, to reduce or even eliminate the need for large amounts of explicitly labelled image training data, thus helping to make the most of the available text. For example the first text passage could be an impressions section of a pre-existing report on a medical image (e.g. x-ray or MRI scan, etc.), and the second text passage could be a findings section of the same report discussing the presence or absence of the same potential medical condition in the scan. Or as another example, the first text passage could be a pre-existing medical report and the second text passage could be an augmented or translated version of the same medical report, for example translated from one natural language into another (e.g. French to English), or rewritten with different phrasing or language. Because the first and second passages relate to the same matter, applying contrastive learning between the embeddings (i.e. encoded latent representations), it is possible to provide for some initial pre-training of the text model without the need for deliberate, after-the fact labelling of the existing text passages. Then in the subsequent joint-training with the image model, contrastive learning is also used. Thus overall, the disclosed method can make the most of information extracted from pre-existing text passages to provide for a good performance without the need for separate, explicit labelling of the input images or text. This provides a more efficient, scalable approach to training since large amounts of manually labelled training data labelled by a human expert are rarely available, and to obtain such data would often be burdensome, for example requiring a domain expert to churn through many historical images on screen and thus consuming large amounts of computer resource in terms of time and energy.

In embodiments the pre-training may include any one or more of the following.

The first training may additionally comprise: learning a vocabulary of commonly occurring tokens from a corpus of vocabulary training text, each token comprising a letter, subword, word or phrase; and training the text model based on the learned vocabulary.

In some such embodiments said training of the text model based on the learned vocabulary may comprise language masking modelling, MLM. In this case the MLM comprises: masking one or more parts of at least one piece of MLM training text and thereby producing masked MLM training text; inputting the masked MLM text to the text model and thereby generating, based on the tokens from the learned vocabulary, a token embedding for each of a plurality of instances of the tokens found in the masked MLM text; and predicting at least one of the one or more masked parts of the MLM training text based on at least some of the token embeddings found in the masked MLM text, and based thereon providing a feedback signal to the text encoder to train the text encoder.

In further embodiments, the first training (i.e. the pre training of the text model) may be performed over a first phase, a second phase following the second phase, and a third phase following the second phase. The first phase may comprise the learning of the vocabulary, the second phase may comprise the MLM, and the third phase may comprise the training based on the first and second text passages (inputting the first and second text passages and said training of the text model to minimize the statistical distance between the value of the first and corresponding second text embedding).

Such pe-training techniques improve the computational efficiency of the overall training, and are thus able to achieve a greater performance for a given number of processing cycles during training compared to other multi-modal analysis models such as GLoRIA and ConVIRT, or equivalently achieve a comparable performance in fewer processing cycles of training.

In embodiments, each image may be divided into a plurality of spatial units; wherein for each image input to the image model, the image model may be configured to generate a local embedding per spatial unit of the input image. In this case the generating of the respective image embedding for each image may comprise combining the local embeddings of the respective image into the respective image embedding.

In some such embodiments, the method may comprise, after the first training and joint training: receiving a text query and a target image; inputting the target image into the image model and thereby generating a local embedding per spatial unit of the target image, and inputting the text query into the text model to generate a corresponding text embedding; comparing the text embedding of the text query with each of a plurality of the local embeddings of the target image and thereby linking the text query to a region of the target image; and outputting graphical data for rendering, on a display, graphical information indicating the linking between the region of the target image and the text query.

By training an encoder to generate local (i.e. per spatial unit) embeddings, and then keeping the spatial granularity in the latent space when processing actual text queries in the deployment stage, advantageously the model does not require an object recognition or segmentation module to be attached to the output of the image encoder as in GLoRIA and ConVIRT, thus achieving further a computational efficiency. In embodiments the presently disclose model comprises no object detection module or image segmentation module.

In embodiments each of the spatial units comprises a respective plurality of pixels of a respective sub-area of the image, and the generating of each local embedding comprises combining the respective plurality of pixels using a dilated convolution operation.

This yields a more precise object localization compared to using a pooling operation with an image encoder.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Nor is the claimed subject matter limited to implementations that solve any or all of the disadvantages noted herein.

**Brief Description of the Drawings**

To assist understanding of the present disclosure and to show how embodiments may be put into effect, reference is made by way of example to the accompanying drawings in which:

Figure 1 is a schematic block diagram of a system for scanning subjects and generating reports in accordance with embodiments disclosed herein,

Figure 2A is a flow chart of a method of training a machine learning model in accordance with the present disclosure,

Figure 2B is a flow chart of a method of using a trained machine learning model in a deployment stage in accordance with embodiments disclosed herein,

Figure 2C is a flow chart of a method of generating reports in accordance with embodiments disclosed herein,

Figure 3A is a schematic mock-up of an example user interface in accordance with embodiments disclosed herein,

Figure 3B is another schematic mock-up of an example user interface in accordance with embodiments disclosed herein,

Figure 4 is a schematic representation of a multi-modal machine learning model comprising a text model and an image model in accordance with embodiments disclosed herein, and

Figure 5 is a schematic representation of a text model in a pre-training phase in accordance with embodiments disclosed herein, and

**Detailed Description of Embodiments**

The following describes new machine learning techniques for making the most of text semantics to improve multi-modal image analysis in fields such as biomedical vision-language modelling. For example, the disclosed techniques may be used to provide for better modelling of clinical text data to supervise a medical image analysis model, and localization of clinical findings in x-ray scans may be extracted for "free" (i.e. without the need for explicit labelling) as part of the disclosed model design and training. Input image models can be better utilized through input text prompts (queries), which significantly reduces the need for costly manual annotations and the computational cost that would otherwise be incurred by additional classification or localization networks. Further, both text and image models can provide supervision to each other in a bi-directional manner to achieve better downstream task performance.

As mentioned previously, multi-modal data such as radiology images and corresponding reports are often widely available in fields such as medicine. It would be desirable to make more efficient use of such data. For example, interpreting this data at scale will be beneficial for improving clinical care and accelerating clinical research. Text with complex semantics such as biomedical text poses additional challenges in vision-language modelling, and previous work has used insufficiently adapted models that lack domain-specific language understanding. The present disclosure show principled textual semantic modelling can substantially improve contrastive learning in self-supervised vision-language processing. There is disclosed a language model that achieves good results in natural language inference through its improved vocabulary and novel language pretraining objective, leveraging semantics and discourse characteristics in textual training data such as radiology reports. Further, there is disclosed a self-supervised joint vision-language approach with a focus on better text modelling.

Embodiments may provide any one or more of the following aspects.

I. A novel text model pretraining strategy that performs text-only self-supervised learning to learn better sentence representations. This includes contrastive learning with text, and in embodiments may also comprise masked-language-modelling (MLM), and domain-specific vocabulary extraction.

II. Better text models and sentence embeddings enable new ways of utilising joint image-text models for multiple downstream applications. This may provide for object detection, segmentation, classification and grounding tasks without requiring any additional model training, solely by re-utilising the joint latent space. This aspect may achieve reduced computational demand and labelling efforts, thanks to novel use of good quality text sentence encodings at inference time with multiple text queries.

III. A new way of re-using text models as classifiers at test time require less training compute and manual labels.

IV. The spatial resolution of image patch embeddings is enhanced by using dilated convolutions rather than pooling operations. Dilated convolutions yield more precise object localisation and grounding performance, since pixels can be encoded in a denser fashion.

V. The use of multiple text queries which yield better object or abnormality detection performance in the images. Additionally, rather than aggregating projected image embeddings into a single vector, in embodiments the image spatial grid is maintained both at test and training times.

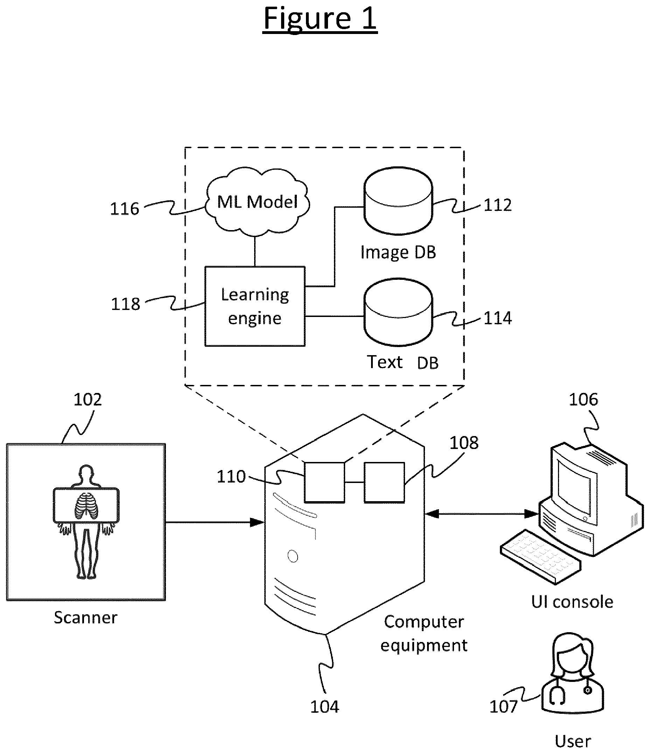

Figure 1 shows an example system in accordance with embodiments of the present disclosure. The system comprises at least one image source (e.g. image capturing device) 102, computer equipment 104, and at least one user interface (UI) console 106. The at least one image source 102 and the UI console 106 are operatively coupled to the computer equipment 104, either directly or via one or more networks, such as the Internet, a mobile cellular network, and/or a private wired or wireless intranet of an organization (e.g. hospital, health organization or research institute). It will be appreciated that any data, signals or information described below as being exchanged between the image source 102 and the computer equipment 104, and between the computer equipment 104 and the UI console 106, may be conducted by any such means of connection and/or others.

The at least one image source 102 may comprise a scanner for producing scans of at least a bodily part of a human or animal subject, living or dead, based on one or more scanning media types. For example the at least one scanner 102 may comprise an x-ray based scanner for producing x-ray scans, a PET scanner for producing PET scans, an MRI scanner for producing MRI scans, an ultrasound scanner for producing ultrasound scans, and/or scanning electron microscopy equipment for producing electron microscopy based scans. Another example could be visible light photography equipment for producing scans in the form of photographs. In embodiments the system may comprise multiple types of scanner for producing multiple types of scan of a given subject based on different scanning media (e.g. x-ray and MRI, x-ray and PET, PET and MRI, etc.). Alternatively only a single scanner employing a single type of scanning medium may be used, e.g. x-ray only, or MRI only, etc. Note also that for a given scanning medium type, there may be different options from producing an image. For example an x-ray based scan as referred to herein could refer to an x-ray photograph or a more sophisticated form of scan based on x-rays as the probing medium, such as a CT (computed tomography) scan. Or a visible light based scan could refer to a simple macroscopic visible light photograph or an optical microscopy based scan.

The following will be described in terms of vision-language processing of bodily scans obtained by an image source 102 in the form of an image scanner such as a medical scanner. However it will be appreciated that this is not limiting. In other use-cases, the images could be any images from any image source 102, such as one or more cameras or an image database of another system. Any mention of scans in the Detailed Description could be replaced more generally with any set of one or more images, and the reports could comprise any pieces of text describing the presence or absence of beings, objects or phenomenon in the corresponding images.

In the case of bodily scans or the like, each individual scan may comprise one or more images. In the case of multiple images per scan, the scan may for example comprise a 3D "stack" of 2D images ("slices"), each image comprising a cross-section through the subject taken at a different respective depth. For instance, while a simple x-ray photograph may comprise only a single x-ray image, a more sophisticated x-ray technology such as a CT (computed tomography) scan may comprise a stack of images representing slices through the subject at different depths, thus creating a tack of 2D images representing a 3D volume of at least a portion of the subject's body. The scanning equipment 102 may be used to take a 2D or 3D scan of a given subject on a single occasion or multiple such scans taken on multiple different respective occasions (different times), e.g. spaced apart by hours, days, weeks, months or years.

The computer equipment 104 may comprise a single computer unit in a single housing, or multiple units in separate housings at one or more sites and networked together using any suitable network technology. For example the computer equipment 104 may comprise one or more server units. E.g. the computer equipment 104 may comprise multiple server units in the same rack, or different units or racks in the same room, different units or racks in different rooms of the same facility or data centre, and/or different facilities or data centres at different geographic sites. In the case of multiple computer units (e.g. server units), these may for example be networked together via a server area network, local area network, intranet, campus area network, metropolitan area network and/or a widearea network or internetwork such as a mobile cellular network and/or the internet.

The UI console 106 may be integrated into one or more of the computer units of the computer equipment 104, and/or may comprise one or more separate computer terminals or dedicated UI peripherals. The UI console 106 comprises at least one display screen, and at least one form of user input means (e.g. a touch screen and/or point-and click interface of the display, and/or a keyboard or voice interface). The UI console is thus able to provide a user interface (UI) for outputting the images of the scans to a user 107, receiving text from the user 107 for writing reports based on the images, and displaying the text of the reports and associated suggestions generated by the computer equipment 104 based on the original text and image(s). The user 107 may comprise for example a medical practitioner, veterinarian, or a researcher. The user 107 may comprise a single such person or a team of two or more people.

The computer equipment 104 comprises processing apparatus 108 comprising one or more processors, and memory 110 comprising one or more memory units. The processor, or each processor, may take any suitable form such as a CPU (central processing unit), or a dedicated AI accelerator processor, or some other, re-purposed form of application-specific processor such as a GPU (graphics processing unit), crypto-processor or DSP (digital signal processor). The memory unit, or each memory unit, may take any suitable form, e.g. an electronic memory medium such as a SSD (solid state drive), flash memory, ROM (read only memory), EEPROM (electrically erasable and programable ROM), RAM (random access memory), SRAM (static RAM), and/or DRAM (dynamic RAM); and/or a magnetic memory such as a hard drive, removable magnetic disk, and/or magnetic tape drive; and/or optical memory such as CD (compact disc), DVD (digital versatile disk), other magnetic disk, quartz glass storage, and/or magneto-optical drive; and/or synthetic biological storage such as synthetic DNA storage.

In some examples, computer executable instructions are provided using any computer-readable media that are accessible by the computing equipment 104. Computer-readable media include, for example, computer storage media such as memory 110 and communications media. Computer storage media include volatile and non-volatile, removable, and non-removable media implemented in any method or technology for storage of information such as computer readable instructions, data structures, program modules or the like. Computer storage media include, but are not limited to, Random Access Memory (RAM), Read-Only Memory (ROM), Erasable Programmable Read-Only Memory (EPROM), Electrically Erasable Programmable Read-Only Memory (EEPROM), persistent memory, phase change memory, flash memory or other memory technology, Compact Disk Read-Only Memory (CD-ROM), digital versatile disks (DVD) or other optical storage, magnetic cassettes, magnetic tape, magnetic disk storage, shingled disk storage or other magnetic storage devices, or any other non-transmission medium that can be used to store information for access by a computing apparatus. In contrast, communication media may embody computer readable instructions, data structures, program modules, or the like in a modulated data signal, such as a carrier wave, or other transport mechanism. As defined herein, computer storage media do not include communication media. Therefore, a computer storage medium should not be interpreted to be a propagating signal per se. Propagated signals per se are not examples of computer storage media. Although the computer storage medium (the memory 110) is shown within the computing equipment 104, it will be appreciated by a person skilled in the art, that, in some examples, the storage is distributed or located remotely and accessed via a network or other communication link (e.g., using a communication interface).

Whatever form it takes, the memory 110 is arranged to store an image database 112, a text database 114, a machine learning model 116, and a learning engine 118. The image database stores the image data from one or more scans taken by the at least one scanner 102. It may also store one or more past or historic scans which may have been taken via the same scanning equipment 102 or other scanners, or a combination. The text database 114 stores text data, such as a reports on the one or more scans taken by the scanner 102, e.g. the reports having been authored by the user 107 via the UI console 106. The text database 114 may also store one or more past or historic reports authored based on the past or historic scans, which may have been authored via the UI of the same UI console 106 or a different UI, by the same user 107 or a different user, or a combination. Alternatively or additionally, the text database 114 may also store other textual training data such as past medical articles or papers, etc., or more generally any text passages comprising suitable vocabulary for learning the language of the domain in question.

The machine learning (ML) model 116 may comprise one or more neural networks, or any other form of statistical model capable of learning based on training data, such as a clustering model. The machine learning engine 118 comprises software arranged to take one or more scans from the image database 112 and one or more associated reports from the report database, and input them to the machine learning model 116 to cause it to make predictions (also called inferences) based on the input image and report data. Particularly, these predictions comprise one or more suggested updates to the report(s), as will be discussed in more detail shortly. The machine learning engine 118 may also comprise one or more machine learning algorithms arranged to train the machine learning model 116 based on past images and associated reports from the image database 112 and report database 114 respectively. Alternatively the model may have been trained elsewhere, on other computer equipment (not shown), and copied to the memory 110.

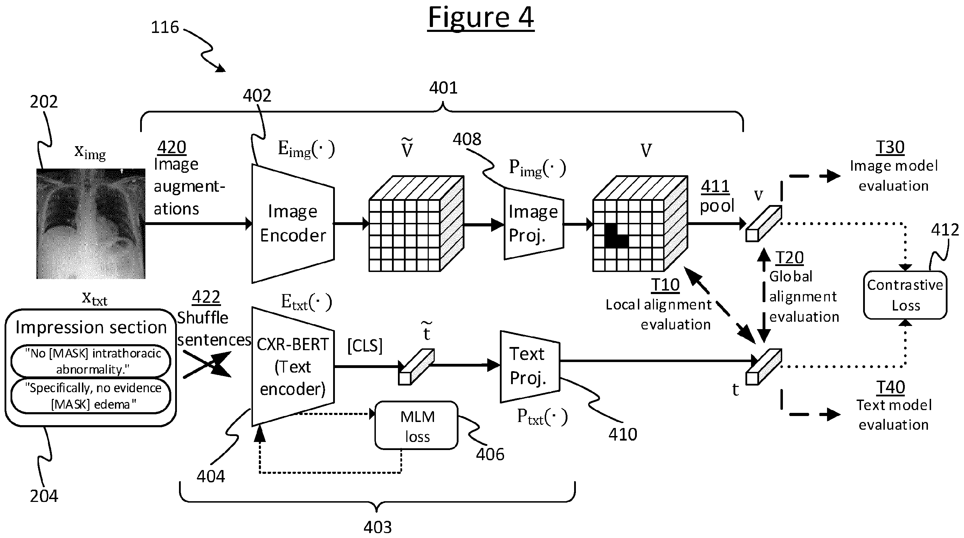

Figure 4 shows an example of the ML model 116 in accordance with embodiments of the present disclosure.

The ML model 116 comprises an image model 401 comprising an image encoder 402 and an image projector 408. The ML model 116 further comprises a text model 403 comprising a text encoder 404 and a text projector 410. The text model 403 may also comprise a masked language modelling (MLM) block 406. Each of the image encoder 402 and text encoder 404 may comprise one or more neural networks. The projectors 408, 410 and the MLM block 406 may each also comprise a neural network. E.g. in embodiments each comprises a multi-layer perceptron. The MLM block 406 may be implemented as a Bert language model prediction head. The ML model 116 further comprises a distance function 412, which may also be called a loss function.

The image encoder 402 is arranged to receive input images ($x_{img}$) 202, e.g. in the form of bodily scans such as medical scans showing a possible condition (or lack thereof) of a subject (e.g. patient), as discussed previous. Each input image 202 could be a 2D image or a 3D image (e.g. a static stack of 2D images capturing a volume, or a video in which the third dimension is time). The text encoder 404 is arranged to receive input text ($x_{txt}$) 204. At least some of the pieces of input text will comprise textual reports describing a condition (or lack thereof) of a being or object shown in the input images 202, e.g. a report of a possible medical condition in a bodily scan. More generally a textual report as referred to here could refer to any one or more passages of text that describe (i.e. report on) a respective input image 202.

Other pieces of input text input to the model 204, e.g. during training, could include passages not directly reporting on a particular input image 202 but rather comprising examples of text on the topic that the ML model 116 may be trained to handle, e.g. comprising medical language describing a particular medical matter. Such input text 204 may be used in an initial pre-training stage to train the text model 403, as will be discussed in more detail shortly.

The image encoder 402 is arranged to encode each input image 202 into at least one respective embedding. Preferably the image encoder 402 is arranged to encoder each input image 202 into a respective matrix $\tilde{V}$ of local embeddings, one local embedding for each different spatial unit (or "patch") in a grid of spatial units into which the respective image 202 is divided. For example each spatial unit of a given image 202 could be a different individual pixel, or a different block of pixels within the image (e.g. 8x8 pixel block); in which case the respective matrix of local embeddings $\tilde{V}$ comprises an individual local embedding encoding each pixel or block of pixels in the respective image 202. The local embeddings may also be referred herein to as "patch embeddings", i.e. an embedding per "patch" (per spatial unit) of the input image 202. Each individual local embedding is itself a vector which encodes the information in the respective spatial unit or patch of the image.

The text encoder 404 is arranged to encode each of one or more text portions in the input text 204, e.g. each clause, sentence, paragraph or whole document, into a respective text embedding $\tilde{t}$. E.g. the text encoder 404 may encode each of one or more linguistic units or subdivisions of the input text, such as each clause, sentence or paragraph, into a respective text embedding. Or the text encoder 404 may encode an entire passage or document into a single text embedding. Either way, the text embedding encodes the information in the respective portion of text.

An embedding is a representation of the information from the input feature (in this case the input text 204 or image 202) in compressed, numerical form. This is also sometimes referred to as a "latent vector". The image model 402 learns to encode the images 202 into the image embeddings $\tilde{V}$, and the text model learns to encode the text from the reports 204 into the text embeddings $\tilde{t}$. The image embedding captures a kind of "summary" of the visually relevant information content in the image data, and the text embedding captures the semantically relevant information in the text. The concept of an embedding or latent vector will, in itself, be familiar to a person skilled in the art. Each of the image model 402 and the text model 404 may take the form of a neural network arranged as an encoder network. Encoders for encoding image and text data into latent space are, in themselves, known in the art.

The image projector 408 and the text projector 410 are arranged to project the image embeddings $\tilde{V}$ from the image encoder 402 and text embeddings $\tilde{t}$ from the text encoder 404, respectively, into a common latent space, i.e. such that the resulting latent vectors $\tilde{V}$, t from the image and text branches of the ML model 116 are the same length as one another.

A projection converts an embedding into another embedding for the purpose of "alignment", i.e. combining between different modalities (in this case images and text). So the unprojected version of the image embeddings $\tilde{V}$ are converted into projected versions V, and the unprojected version of the text embeddings $\tilde{t}$ are converted into a projected versions t. This means the dimensionality of the latent vector of one or both of the image and text embeddings is scaled such that they have the same dimensionality for the purpose of comparison. The raw embedding $\tilde{V}$, $\tilde{t}$ as immediately output by each of the image and text models 402, 404 (respectively) is optimized for compression, whilst the projected embeddings V, t are optimized for alignment, i.e. aligning between different modalities (in this case images and text). The projection operation bridges between the two latent spaces. It converts the embedding to a different latent space in order to decouple alignment from compression.

However, in alternative embodiments, it is not essential to employ projectors 408, 410 separate from the encoders 402, 404. Instead the image encoder 402 and text encoder 404 could be configured to encode directly into a common latent space for alignment. Or in another variant, only the image branch may be equipped with a projector 408, so as to scale the image embedding to match the dimensionality of the text embedding as inherently output by the text encoder 404; or vice versa, only the text branch may be equipped with a projector 410, so as to scale the image embedding to match the dimensionality of the text embedding as inherently output by the image encoder 402.

In the case of generating local embeddings per spatial unit of the image 202, the ML model 116 may also comprise a combining function 411 which combines the individual local projected embeddings of a given input image 202 into a combined ("global") embedding v. The combining function 411 may comprise a pooling function arranged to combine the local embeddings of a given image 402 by pooling.

As another alternative, in less preferred embodiments, the image encoder 402 could be configured to encode directly into a single global embedding per input image (without local spatial embeddings), in which case a separate combining function 411 is not needed. Such an embodiment would not retain the local (i.e. per spatial unit) information required for alignment between text and specific image regions, as will be discussed in more detail shortly. Retaining the ability for alignment is preferred but is not essential to all possible use cases that could be envisaged for the training techniques disclosed here (e.g. instead the trained model 116 could be used to evaluate statements made about an image as whole or to make predictions from a whole image).

In a joint training stage (discussed later), the projected, global version of the image embeddings v and the text embedding t are input into the distance function 412, e.g. a contrastive loss function. The distance function 412 is a multi-modal learning function such as a contrastive loss function which determines the value of a multi-modal distance metric - also called a loss metric. Alternatives to a contrastive loss function include margin based loss functions, and ranking based loss functions. Distance metrics suitable for multi-modal learning, including vision-language processing, are in themselves known in the art.

The loss function evaluates the distance metric (e.g. contrastive loss), which gives a measure of loss between the global embedding v of the input images 202 and the corresponding embeddings t of textual reports 404 describing the respective images. This may be used to train the image and text models 402, 404 to minimize the average or overall loss over a plurality of image-report combinations, as will be discussed in more detail shortly.

In the case of using encoders 402, 404 and separate projectors 408, 410, the encoders learn 402, 404 learn to compress the image and text data, respectively; and the respective projectors 408, 410 learn to project each of the image and text embeddings into a respective projected embedding which is optimized to align between the two modalities. The concept of projecting an embedding for the purpose of alignment between modalities in machine learning is, in itself, known in the art. An advantage of learning to encode an input vector into a first embedding optimized for compression, which is then projected into a projected embedding for alignment, is that the image model 402 and/or text model 404 can initially be trained independently of one another based on only image and/or text data (respectively). That is, the image model 402 may be part trained based on images that have no corresponding report, and/or the text model 404 may be part trained based on text data for which no corresponding images have been kept. The constituent models 402, 404 can then be combined via the projected embeddings V, t and distance function 412 and further trained based on combined image and report data (reports for which the respective image data is still available). However, this is not essential and in other embodiments the image model 402 and/or text model 404 may be configured to encode directly into a respective embedding V, t optimized for alignment. In that case the contrastive loss function 412 may be replaced for example with a binary contrast estimation function.

Other possible approaches to multi-modal machine learning include noise contrastive estimation and image-text matching loss.

Whatever form the ML model 116 takes, the learning engine 118 may manage the input of training data into the model 116. Over many training data samples, the learning engine 118 trains the model 116 by tuning the parameters (e.g. weights) of the model 116 based on the output, e.g. by a stochastic gradient descent algorithm. Details of algorithms for training a machine learning model such as a neural network based on back propagation or the like will, in themselves, be familiar to a person skilled in the art.

In embodiments, the image model 401 further comprises an image augmentation function 420 arranged, at least during some of the training, to apply one or more image augmentations (i.e. transformations) to the input images 402 before they are input to the image encoder 402. These one or more augmentations could comprise, for example, adding noise, or random rotations or lateral translations. When trained based on image data including such transformations, this helps make the image model 402 more robust in the deployment stage and prevents over fitting. E.g. the model will be better able to handle images in which a target body part of the patient is rotated or translated.

As an alternative or additional augmentation, the text model 403 may comprise a sentence shuffling function 422 arranged, at least during some of the training, to shuffle one or more sentences in the input text 204 before being input to the text encoder 404. This may comprise swapping the order of a pair of sentences, or moving one or more sentences to another position and shifting other sentences accordingly. Sentence shuffling advantageously improves the robustness of the text model 404 and helps prevent over fitting.

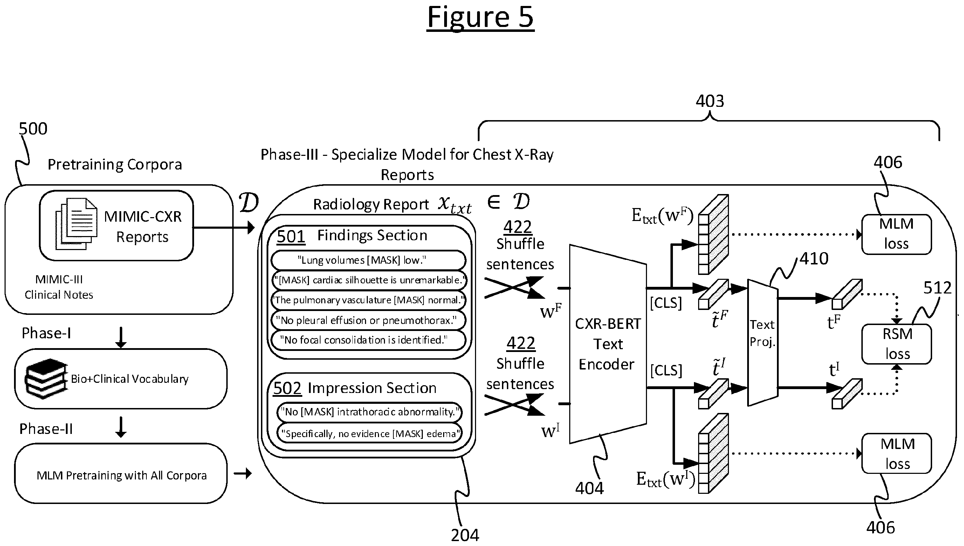

As yet another alternative or additional feature, the ML model 116 may comprise the MLM block 406 for performing masked language modelling, as will be discussed in more detail later with respect to Figure 5.

Figure 4 also illustrates some optional evaluation steps T10, T20, T30, T40 which may be performed to evaluate the ML model 116 in some embodiments of the disclosed method.

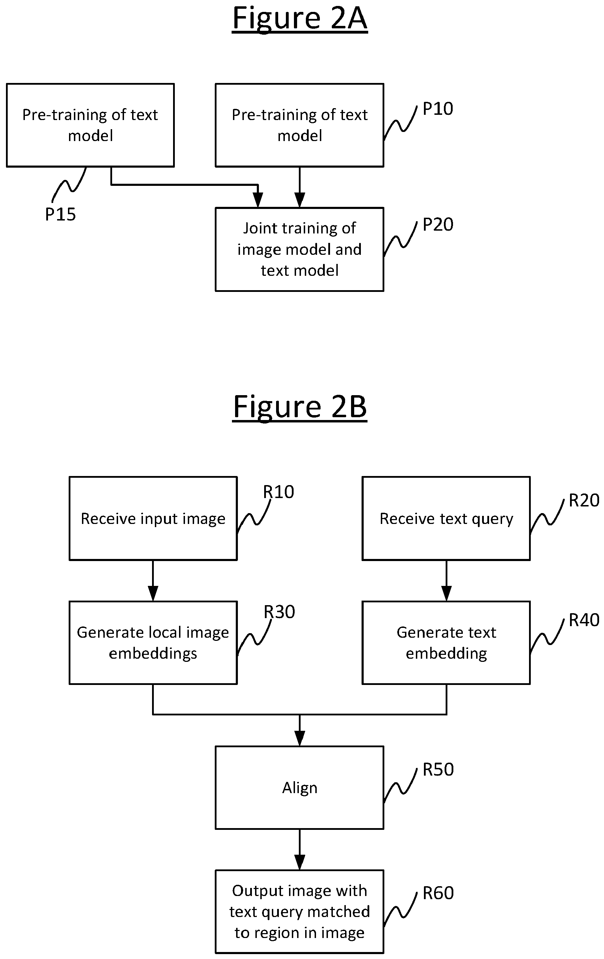

Figure 2A shows a method of training the ML model 116 in accordance with the present disclosure. Corresponding detail of an example implementation of the text model 403 is shown in Figure 5.

At step P10 the ML engine 118 performs a pre-training stage whereby it trains the text model 403 independently of the image model 401. The pre-training is based on a corpus of text training data 500 from the text database 114. The text training data 500 comprises a plurality of text passage combinations. The ML engine inputs each of a plurality of the text passage combinations into the text model 403 from the text database 114. Each text passage combination comprises a respective first text passage 501 and a corresponding second text passage 502 describing a same target matter (e.g. same type of entity or same topic) as the respective first text passage. In other words, each text passage combination comprises two differently worded descriptions of the same thing. Each text passage combination thus provides a text training data point for training the text model 402 using contrastive learning, a form of unsupervised learning. Each passage is an instance of the earlier-described text 204 that may be input to the text model 403. A given first or second passage 501, 502 may be of any length, whether one or more sentences, one or more paragraph, one or more sections of a document, or even each being a whole document or set of multiple documents.

The target matter described by both the first and second text passage of a given text passage combination could be a type of physical entity such as a species of living being or a type of object. Or it could be a particular condition or feature that may be found in a physical entity, such as a medical condition that may be experienced by a species of living being, or particular limb or organ of a living being; or a physical condition or feature that may be found in a type of object such as a particular type of electrical, electronic or mechanical device; or a composition or other state that may be found in a type of substance such as a chemical substance or manufactured substance. Or the target matter could be a particular topic or subject, e.g. a certain medical condition, weather phenomenon, etc.

In one example use case, each text passage combination describes a condition (e.g. disease, illness or other such medical condition) that may be experienced by a species of living being such as a human or animal. This can be used to help train the text model 403 for use in medical diagnostics or other such analysis of a condition of the species in question. In another example use case, each text passage combination may describe a condition of a physical object or substance such as a particular type of mechanical device, electronic device, electrical device, manufactured substance or object, or chemical composition. This may be used to help train the text model 403 for use in diagnostics or other such analysis of devices, such as to diagnose a problem with a mechanical, electrical or electronic device; or to analyse the state or composition of a substance or chemical composition; or to analyse the structural state or physical condition of an object, e.g. to detect a state of wear, damage or disrepair of an object. In yet another example, each text passage combination may describe physical features of a species of living being or a type of object, in order to train the text model 403 for use in image recognition, such as to detect the presence or absence of a being or object in images, e.g. for the purpose of presence detection or such like.

The first and second text passage 501, 502 of a given text passage combination could be different passages (e.g. sections) within the same document. For example the first text passage 501 could be a findings section of a report (e.g. medical report) on one or more bodily scans of a subject (e.g. patient), and the second text passage 502 could be an impressions section of the same report. Or the first text passage could be an abstract of a document and the second text passage could be (at least part of) the body of the same document. Alternatively the first and second text passages 501, 502 could be found in different documents. E.g. the first text passage 501 may describe the target matter in a first natural language (e.g. English) and the corresponding second text passage 502 may describe the same matter in a second natural language different than the first (e.g. French). The second text passage could be a human or machine translation of the first or vice versa, or both could be a human or machine translation of the same source material authored in a third natural language. As yet another example, the first and second text passages 501, 502 could be different descriptions of the same matter by different authors. Or as yet another alternative or additional example, one or both of the first and second text passages 501, 502 could be subject to one or more artificial, automated text augmentations, such as sentence shuffling, masking of words, or other artificially generated errors or omissions.

For each text passage combination that is input into the text model 403, in response the text encoder 404 encodes the first text passage 501 into a respective first text embedding $\tilde{t}^F$, and encodes the second text passage 502 into a second text embedding $\tilde{t}^I$. Each of these is input to a loss function 512 of the text model 403. In an example use case this may be referred to herein as a radiology section matching (RSM) function. It is another instance of a loss function, e.g. contrastive loss function, and may work similarly to the loss function shown in Figure 4 which is used for the joint training of the image and text models 401, 403. In embodiments the first and second text embeddings $\tilde{t}^F$, $\tilde{t}^I$ are input to the text model's loss function 512 via the text projector 410 such that the first and second text embeddings are input in the form of respective projected versions $t^F$, $t^I$. Alternatively the projection by the text projector 410 is not necessary at this stage since the output of the text encoder 404 may already be configured to output the embeddings of the first and second passages 501, 502 with the same dimensionality for each text passage irrespective of length.

Either way, the distance function 512 is configured to evaluate a measure of overall difference (the "loss") between the embeddings of the first text passages 501 as compared to their corresponding second text passages. To do this, the loss function 512 determines a measure of the pairwise distance, according to a suitable distance metric, between the embedding of the first and the embedding of the corresponding second text passage 502 for each data point (each text passage combination) in a batch of data points. The distance metric (which could also be called a similarity metric) quantifies the similarity or different between two samples or instances. The distance metric could be e.g. the cosine similarity or mean square distance. One could use same distance metric in preliminary training P10 as used later in joint training P20, or different distance metric. Either way, the loss function 512 tunes the text model 403 (e.g. the weights in the case of a neural network) so as to attempt to minimize a loss metric for the batch. The loss metric gives a measure of average or overall difference or similarity across the batch of data points. This may then be repeated over multiple batches of data points so as to gradually tune the model 403 toward an optimal solution which minimizes the overall loss, e.g. based on stochastic gradient descent. The loss metric may for example be contrastive loss, which puts the pairwise distances in context by normalising paired distances with respect to each other. The normalized distances are relative distances, e.g. quantifies how close a given point is to an anchor sample in comparison to a set of data points. The metric used to measure the relative or normalized pairwise distances could be for example infoNCE (information noise contrastive estimation) or triplet loss. The metric used to measure overall loss across the batch could be for example negative log likelihood.

Whatever metrics are used, the ML engine 118 is configured to train the text model 403 to minimize a measure of average or overall difference between first and second text passages statistically over the multiple training data points provided by the multiple text passage combinations. For example this may comprise tuning the weights in the case where thee text model 403 comprises one or more neural networks. The training comprises training (e.g. tuning the weights of) at least the text encoder 404. In embodiments it may also comprise training the text projector 410.

The multiple text training data points (i.e. the different text passage combinations) may be input though the text model 403 in series, one after another. Alternatively they may be input in parallel through different parallel instances of the text model 403. Or a combination of series and parallel processing may be employed. Note also that minimize or optimize in the context of an iterative statistical method such as machine learning does not necessarily mean finding the absolute possible minimum or a global minimum. Rather, more broadly it may mean, to a degree practically achievable over the finite iterations that can be performed using the training data samples available, tending towards an estimated global or local minimum or a reduced measure of overall distance (i.e. loss) compared to a starting state of the model.

In embodiments the sentence shuffling 422 is applied to one or both of the first and second text passages 501, 502 in one, some or all of the text passage combinations before input into the text encoder 404 in the pre-training stage P10.

Optionally, a step P15 is also performed in which the image encoder 404 can also be pre-trained prior to joint training (step P20, discussed shortly). Pre-training of image encoders per se is know in the art. For example this could be done using SimCLR contrastive learning. This does not require any manual labels on images, and it is performed by contrastive learning between augmented versions of images (e.g. chest x-ray images). As the image and text pretraining are independent of one another, step P15 could be performed before or after P10, or partially or wholly in parallel.

Once at least the text model 403 has undergone at least some pre-training at step P10, and optionally also after some pretraining of the image model 401 at step P15, then the method then proceeds to step P20 where the ML engine 118 performs joint training of the image model 401 and text model 403 together. This comprises a similar form of contrastive learning as used in the text training stage P10, but now between image and text embeddings v, t.

In the joint training, the ML engine 118 inputs each of a plurality of image-text combinations into the ML model 116. Each image-text combination comprises a respective image 202 and a corresponding textual (text-based) report 204 describing the respective image (meaning it describes at least some aspect visible in the respective image 202, not necessarily describing everything in the entire image). Again each image may take the form of a 2D image or 3D image (e.g. a stack of 2D images representing a volume, or a video). Each report 204 may or may not comprise some or all of one or both of a respective pair of first and second text passages 501, 502 that were used in the pre-training stage P10. In embodiments, the reports 204 of at least some of the image-text combinations used in the joint training P20 comprise at least one of the first and second passages 501, 502, or part thereof, from a respective text passage combination used in the pre-training P10. I.e. at least some of the same text is used in both the pre-training P10 and the joint training P20. For example reports by medical practitioners on actual medical scans could be used both in the pre-training and the joint training. Alternatively or additionally, in embodiments at least some of the reports 204 used in the joint training P20 may comprise different text than used in the first and second passages 501, 502 in the pre-training; or at least some of the first and/or second text passages 501, 502 used in the pre-training P10 may comprise text not found in the reports 204 used in the joint training stage P20. For example the first and/or second text passages 501, 502 used in the pre-training P10 could comprise text from sources such as medical journals or academic papers which do not describes specific instances of the medical condition in specific images. As another example the first and/or second text passages 501, 502 used in the pre-training P10 could comprise reports from personal examinations of a subject by a practitioner which did not involve a scan and hence do not have associated images.

Either way, each textual report 204 used at the joint training stage P20 comprises text describing something about the respective corresponding image 202 of the respective image-text combination. Thus each image-text combination provides a respective training data point or use in the contrastive learning of the joint training stage P20.

By way of example, in the case of a bodily scan such as in the medical use case, each image 202 may comprise a medical scan (e.g. X-ray, MRI, etc.) of a human or animal subject or at least a certain body part of the subject, as discuss earlier with respect to Figure 1. Each corresponding report 204 may comprise text describing an occurrence of a certain condition

(e.g. medical condition such as a disease or illness) as visible from the respective image. In another example use case, for the diagnostics or analysis of a physical object or substance, such as the diagnostics of an electrical, electronic or mechanical device, or the analysis of the state of an object or chemical substance, then each image 202 may comprise an image of a specimen of at least part of the type of object or substance in question. Or in an image recognition scenario, each image 202 may comprise an image of a scene in which a member of the species or a specimen of the object to be detected may potentially be present.

The corresponding report 204 may comprise a portion of text of any length, whether one or more sentences, one or more paragraphs, one or more sections of a document, a whole document, or more than one document. Whatever the length, it describes something about what is visible in the respective image 202. E.g. in the case of a bodily scan, the corresponding report makes a statement about the possible presence or absence of a condition of the subject based on the respective image. Or for the diagnostics or analysis case, the corresponding report makes a statement about the state of the object or substance in question as visible from the respective image. Or for the image recognition use case, the corresponding report makes a statement about the possible presence or absence of the type of being or object in question.

Whatever the application scenario, for each image-text combination, the ML engine 118 inputs the respective image into the text encoder 404 and the respective report into the text encoder 404. For each image 202 that is input, in response the image encoder 402 encodes the image into a respective global embedding v. In embodiments this may be done by first encoding into a local embedding per spatial unit ("patch") of the image in a grid of spatial units into which the image is divided, and the combining the local embeddings via the combining function 411 (e.g. by pooling) as discussed previously. Also, in embodiments each local embedding may be projected into a projected version by the image projector 408 before the local embeddings are combined into the global embedding v, as also discussed previously.

For each report 204 that is input, in response the text encoder 404 encodes at least part of the report into a respective text embedding t. As discussed previously, in embodiments this is done by encoding into an unprojected version $\tilde{t}$ and then passing through the text projector 410 to produce a respective projected version t.

For each image-text combination, the respective image embedding v and the respective text embedding t for the report is input to the loss function 412 to evaluate a metric which measures the pairwise distance or similarity (i.e. the difference) between the image embedding and the corresponding text embedding, e.g. cosine similarity or mean square error, for each of a plurality of data points (i.e. image-text combinations) in a batch. Based on this, over multiple data points (i.e. multiple image-text combinations), the ML engine 118 trains the image model 401 and the text model 403 together to try to minimize a measure of the average or overall loss statistically over the multiple image-text combinations of the batch. This may then be repeated over multiple batches. The same types of metric may be used as described earlier in relation to the pre-training P10, e.g. a contrastive loss metric which measures a normalized similarity over v and t, whereby loss(t, v) = sim(t, v) / sum of all sim(t, v') in a batch. The training of the text model 403 comprises at least training the text encoder 404 (e.g. tuning its weights in the case where implemented using one or more neural networks). In embodiments the training of the text model 403 may also comprise training the text projector 410. The training of the image model 401 comprises at least training the image encoder 402 (e.g. tuning its weights in the case where implemented using one or more neural networks). In embodiments it may also comprise training the image projector 408.

By the use of unsupervised, contrastive learning in the text pre-training P10 and the joint training P20, then this advantageously reduces the need for labelling and the intensive resource usage associated therewith. In embodiments none of the images 202 used in the joint training P20 is manually labelled by a human labeller. And/or, in embodiments none of the text passages 501, 502 used in the pre-training P10 is labelled after-the-fact by a human labeller (i.e. there is no step of going back at a later date to separately label the text passages after the content was authored by the original author). And/or, in embodiments, none of the text reports used in the joint training P20 is labelled after-the-fact by a human labeller.

Further, by first training the text model 403 based on domain specific text examples in a pre-training phase P10 before starting the joint training, this advantageously improves the efficacy of the joint image-text training P20 once it does begin.

The multiple data points used in the joint image-text training (i.e. the different image-text combinations) may be input though the ML model 116 in series, one after another. Alternatively they may be input in parallel through different parallel instances of the model 116. Or a combination of series and parallel processing may be employed. Also, note again that minimize or optimize in the present does not necessarily mean finding the absolute possible minimum or a global minimum, but rather more broadly may refer to tending toward an estimated global or local minimum or a reduced measure of overall distance to a degree practicable given the training data available.

In embodiments, in the joint training stage P20 the text encoder 404 may encode the entire report 204 on each image 202, or a single part of the report, into a single text embedding per image 202, thus generating a single data point per image 202 for use in the joint training P20. Alternatively each report 204 may be broken down into multiple different portions, e.g. individual sections, paragraphs or sentences, and the text encoder may generate a separate individual embedding for each portion. In this case there are multiple text embeddings for each image 202 used in the joint training stage P20, thus providing multiple data points per image for use in the training.

In embodiments the sentence shuffling 422 is applied to the reports 204 in one, some or all of the image-text combinations before input into the text encoder 404 in the joint training stage P20. And/or, in embodiments the image augmentation 420 is applied to the images 202 before input into the image encoder 203 in the joint training stage P20. In embodiments sentence shuffling 422 is used in both the joint training P20 and pre-training P10.

Figure 2B shows a method that may be performed using the machine learning model 116 once trained - in the so-called "deployment" stage or "in-the field", i.e. when actually being used for an application rather than just being trained. Note that describing the model 116 as trained, or being used in the deployment stage or such like, does not exclude that further training could be performed. Indeed, in embodiments the model 116 can keep learning in an ongoing fashion during the deployment stage as well. To say that the ML model 116 has been trained implies only that a certain amount of training has been performed in order to allow it to be used with some reasonable degree of performance.

At step R10, an input image is received into the input of the image encoder 402, and at step R20 an input text query is input into the input of the text encoder 404. The input image is another instance of an image 202 that may be input to the text image model 402 as shown in Figure 4, but this time at the deployment stage rather than a training stage. Similarly the text query is another instance of text 204 that may be input into the text model 403 as shown in Figure 4, but again now at deployment rather than training.

The input image 202 may be selected by a user via a user interface (UI) of the UI console 106. It may be retrieved from the image database 112 or captured from the image source 102, e.g. a scanner or camera. The input image 202 input at the deployment stage may comprise an image of anything that the user 107 wishes to analyse using vision-text processing. For example the input image 202 may comprise a 2D or 3D bodily scan of a human or animal, or a body party of the human or animal, obtained from the scanner 102. Or the image 202 could comprise an image or a physical object or substance which the user 107 wishes to analyse; e.g. an image of an electronic, electrical or mechanical device (or part thereof) which the user wishes to perform diagnostics on; or a physical object (or part thereof) or substance, such as a manufactured object or substance, which the user 107 wishes to assess the state of, e.g. for quality control purposes.

The text query may be entered by the user 107 through the UI of the UI console 107, or may be selected by the user 107 through the UI of the UI console to be retrieved from the text database 114. The text query - which could also be called a text prompt or just input text - may comprise any portion of text 204 input at the deployment stage which comprises one or more propositions about a possible condition or state of the being, object or substance (or part thereof) shown in the corresponding input image 202. For instance, the input text query 204 may comprise a proposition about the occurrence or absence of a possible condition, e.g. a medical condition such as a disease or illness, of a bodily part of a human or animal shown in the image 204. Or the input text query could comprise a proposition about the possible occurrence or absence of a certain condition of an object or substance (or part thereof) shown in the image, e.g. a state of wear or deterioration of the object or substance, or a quality of the object or substance, or a possible fault in the case of an electronic, electrical or mechanical device. As yet another possibility, in an image recognition scenario such for presence detection or object detection, the input text query 204 may comprise a proposition about the possible presence or absence of a member of a particular species (e.g. human) or a particular body part (e.g. human face), or the presence or absence of a particular individual (e.g. particular person or particular person's face), or the presence or absence of a particular type of object (e.g. a vehicle) or a particular instance of an object (e.g. vehicle with a particular license plate). The input text query could be any length, whether one or more sentences, one or more paragraphs, one or more sections of a document, a whole document, or more than one document. Note also that "query" in this context does not necessarily imply that the text in question is phrased grammatically speaking as a question. Rather it could be any proposition phrased as a statement, question or otherwise.

At step R30 the image encoder 402 generates the local image embeddings $\tilde{V}$, for the input image 202. I.e. the image 202 is divided into a grid and an individual latent vector is generated for each unit in the grid (e.g. each pixel or each block of n by n or n by m pixels). In embodiments these are converted into projected versions V by the image projector 408. In the case where the spatial units are larger than individual pixels, i.e. "patches" of pixels (e.g. 8x8 or 16x16 pixel blocks), a simple pooling operation may be used to combine the perpixel information of each patch or block into the respective local embedding. Alternatively however, in accordance with embodiments disclosed herein, a dilated convolution operation may be used instead. This enhances the spatial resolution of image patch embeddings by yielding more precise object localisation and grounding performance, since it can encode pixels in a denser fashion. Pooling refers to aggregating pixel values into a single vector, whereas dilated convolution reduces resolution but still retains some resolution. Dilated convolution per se is a known type of mathematical operation. In embodiments pooling is used at training time and dilated convolution replaces the pooling at the deployment stage (i.e. when the model 116 is being used to process text queries). This is done by replacing the pooling operations present in the image encoder 402 that combine pixel information into patch embeddings. This thus impacts the pixel resolution of the output local patch embeddings V. The pixel representations in V become denser (e.g. 4x) when dilated convolutions is used as compared to pooling, since the resolution of the spatial grid is at least partially preserved. A third option would simply be to use individual pixel embeddings for the local embeddings V, but this would be more demanding in terms of computational resource and memory.

At step R40 the text encoder 404 generates at least one text embedding $\tilde{t}$ from the input text query 204. In embodiments the input query may be broken down into more than one portion, such as separate sentences, clauses, paragraphs or sections, and the text encoder 404 may generate an individual text embedding $\tilde{t}$ for each such. In embodiments each of the one or more text embeddings is converted into a projected versions t by the text projector 410.

Note: steps R10 and R20 could be performed in either order with respect to one another or in parallel. Similarly steps R30 and R40 could be performed in either order with respect to one another or in parallel. In other words it doesn't matter whether the images or text are processed first, or in embodiments they could be processed at least partially in parallel.

At step R50, the ML engine 118 performs a comparison between the local image embeddings generated at step R30 and the one or more text embeddings generated at step D40. In embodiments these will be the projected embeddings V, t as output by the projectors 408, 410. However in other embodiments the compared embeddings could be the raw embeddings $\tilde{V}$, $\tilde{t}$ as immediately output by the encoders 402, 404 (e.g. in the case where both image and text encoder are inherently configured to encode into the same dimensionality latent space). Either way, for each text embedding, the ML engine 118 searches for regions of the image where the text embedding is above a threshold similarity with the local image embedding (the so-called patch embedding). Similarity between embeddings (which are vectors) could be measured using any suitable metric, e.g. cosine similarity or mean square error. This process may be referred to as local alignment or "grounding". The idea is to find a specific region or regions within the image that can be linked to a proposition in the text query.

At step R60, the ML engine 118 outputs data which can be used to control the UI on the UI console to render an indication of the connection between the text query 204 and the linked region of the image 202, or between each portion of the text query (e.g. each sentence) and the linked regions of the image. For example the UI may display the image on screen, and highlight the region or regions in question, or drawing an outline around the region or regions. The text query may also be shown on screen and could be linked to the aligned region or regions e.g. by suitable colour coding, or a graphical or alphanumeric legend mapping the aligned region(s) to the text, or by overlaying the text on the aligned region(s), or linking it by a line rendered between the text and the aligned region(s), etc.

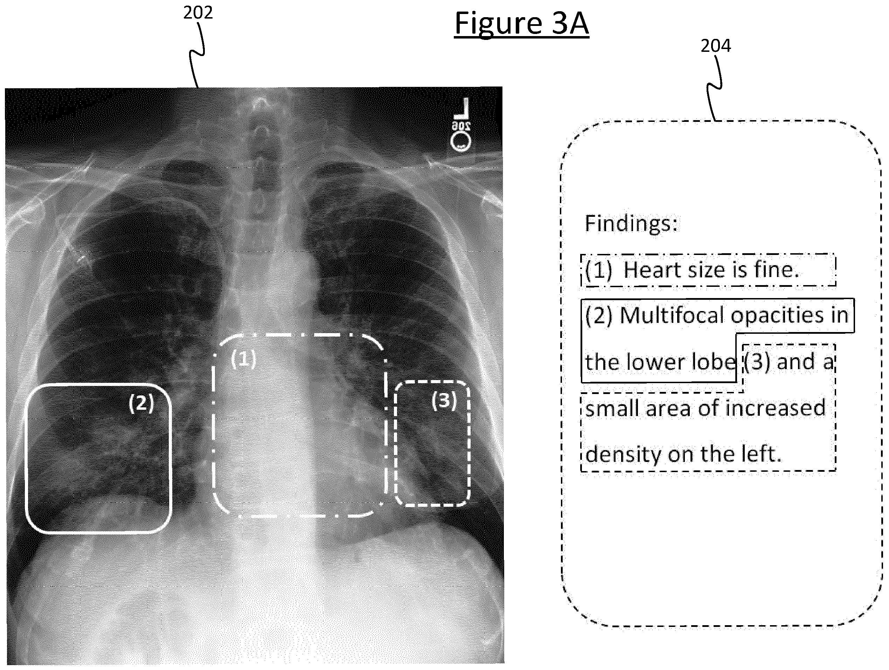

Figure 3A gives an example. In this example the image 202 comprises a bodily scan (e.g. medical scan) of a subject (e.g. patient) - in this case the chest of a human. The corresponding text query 204 comprises a draft report by a medical practitioner such as a radiologist. As separate text embedding has been generated from each of three sentences in the draft report, each making a separate proposition about a possible condition visible in the image. Each of the regions in the image found to align with one of the sentences in the text query is outlined (or instead could be highlighted or such like). Each region may be highlighted with a different colour or outlines with a different outline pattern, and/or marked with a different reference sign (e.g. an alphanumeric sign such as 1, 2, 3). The text query is displayed along side the rendered image, and each sentence is mapped to the type of highlighting or outlining used for a respective linked region in the image, and/or mapped to the reference sign of the respective linked region.

As an alternative or additional use of the ML model 116 once trained, the trained model 116 may be used to automatically generate textual reports on input images 202, or to automatically review human authored reports 204 and potentially make suggestions for updating human-authored report.

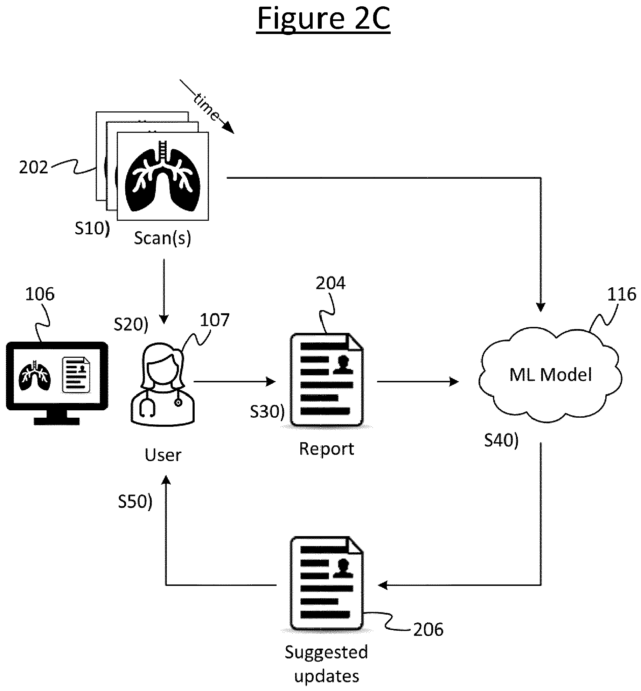

Figure 2C shows an example of a particular method that may be implemented using the system of Figure 1 and model of Figure 4. The method is to be used to estimate a condition (e.g. disease or cause of death) of a target subject. The target subject is either a human or an animal (e.g. mammal, bird, fish, marsupial or invertebrate).

Prior to the method there is a stage of training the machine learning model 116 as discussed previously. The model 116 is trained based on a plurality of past scans (preferably tens, hundreds, thousands or more) of one or more subjects, together with associated reports including text authored based on a review of the respective scans. The past scans preferably comprise one or more of scans at least one subject other than the target subject (but of the same species). They may also include one or more past scans of the target subject. The reports of the past scans may have been authored purely manually, or with AI assistance. They may have been authored by the same user 107 who is to author the new report on the target subject using the method of Figure 2C, or a different user, or a combination. Preferably the past reports include at least some reports authored by different users so as to introduce some variation in style into the training data. Either way, the training may comprise a supervised approach, reinforcement approach or unsupervised approach.

The machine learning model 116 is thus trained to be able to receive the image(s) of a current scan and an associated report, and to output suggestions for updating the text of the report.

At step S10) at least one current scan 202 is taken of the target subject using the at least one scanner 102. As discussed earlier this may comprise for example an x-ray based scan (e.g. CT scan), a PET scan, an MRI scan or an ultrasound scan, or a combination. Each scan 202 may comprise a single 2D image, or a 3D stack of 2D images (e.g. as set of "slices" through the subject).

At step S20), the user (e.g. radiologist, vet or researcher) 107 reviews the one or more images of the current scan 202, or each current scan, though the user interface (UI) displayed on the UI console 106.

At step S30), based on the review, the user 107 authors a draft report 204 (or at least part thereof) - at this stage using his/her professional judgement only (not AI). The report 204 comprises text written by the user 107. The text comprises a description of one or more possible conditions (e.g. diseases) which the target subject may have based on the review. The text may also include an explanation of why the user 107 came to this conclusion, e.g. what features in the image(s) led to this.

At step S40) the image data of the one or more current scans 202 are input into the machine learning model 116, together with at least the text of the corresponding report 204. Based on the report and image(s) together, the machine learning model 116 generates one or more suggestions for amendments 206 to the text of the report 204. For example the suggestions may comprise the addition of one or more potentially missing details, the correction of one or more possible errors, and/or one or more suggestions for alternative terminology (e.g. for clarity or to promote consistency across reports).

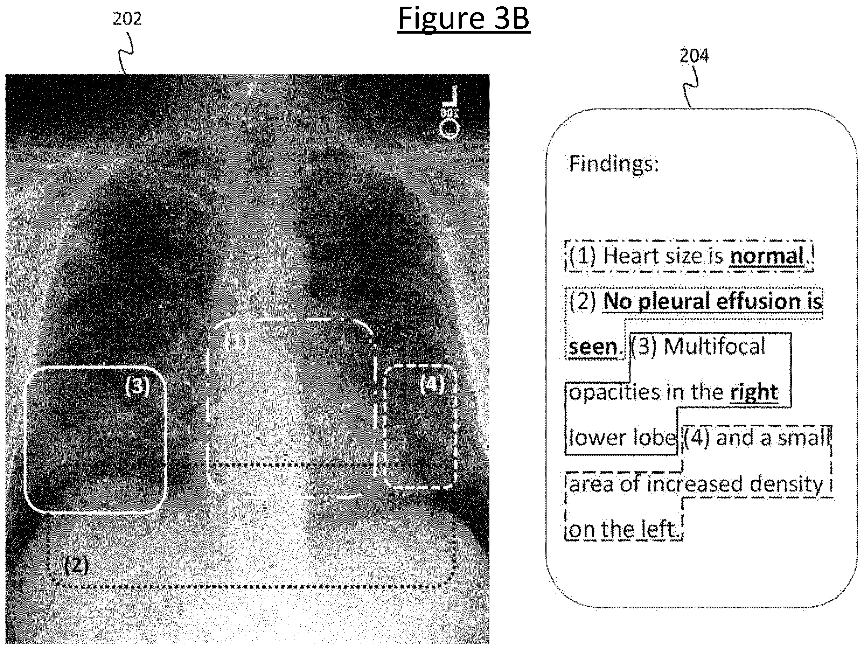

At step S50) the suggested amendments 206 are presented to the user through the UI on the UI console 106. For example they may be presented on screen in the form of a proposed updated version of the report, e.g. with the amendments shown tracked (marked-up) on the previous version of the report (such as by means of strikethrough, underlining and/or highlighting). Further the machine learning model 116 is able to identify which part of the image (or which part of which image) resulted in each suggestion. This is indicated visually to the user 107 through the UI, linking each suggestion to a particular corresponding feature in the image (or one of the images). In other words the UI indicates to the user the cause of each suggestion in the image. Some examples for this will be discussed in more detail shortly with respect to Figure 3B.

The user 107 then reviews each of the one or more suggestions made by the machine learning model 116. However the suggested amendments are not finalized autonomously by the AI. Only if the user 107 deems fit based on his/her professional judgement will he/she choose to accept each update to be included into an actual updated version of the report. The user 107 may accept the suggestions though the UI, for example by selecting to accept or reject each one individually, or selecting accept all or reject all. The selection is made through the UI on the at least one UI console 106, e.g. via a mouse, touchscreen, keyboard or voice input. As another possibility, upon being prompted to reconsider the wording by the suggestion from the machine learning model 116, the user 107 may choose to make an alternative amendment that was neither the original wording nor the suggestion from the ML model 116.

If the user 107 accepts one or more of the suggested amendments, and/or makes one or more amendments of his/her own, then optionally steps S30) to S50) may be repeated with the updated report in order to generate one or more updated suggestions. That is, the amended report (as amended in the first iteration of the method) is input into the machine learning model 116, again along with the corresponding image data of the one or more current scans. The machine learning model 116 processes these together in order to output one or more updated suggestions, which are presented to the user 107, and which the user may again accept or reject, or may make one or more further alternative amendments of his/her own, thus creating a further updated version of the report. In embodiments, the method may continue over one or more yet further iterations in a similar manner, each time the ML model 116 analysing the previously updated version of the report.

Note the method shown in Figure 2C may be somewhat schematized compared to the actual implementation. For instance, in embodiments it is not necessary for the user 107 to compose the entire report 204 and then input it as a whole into the model 116 before the suggestions 206 can start to be generated. In embodiments, the suggestions could be generated and output to the user 107 live as he or she types the report. I.e. the suggestions are output dynamically, or "on the fly", in real-time as the user types, with one or more suggestions being generated and output to the user 107 based on the report as composed so-far before the user 107 has finished writing the whole report.

In embodiments the training of the machine learning model 116 may also be refined with each iteration of the method, i.e. based on the user accepting or rejecting the model's suggestions in each of the one or more rounds of the method.

In some scenarios, then at some point after at least one iteration of the method, one or more additional scans of the target subject may be performed. This adds new image data into the system as well as updated report data. For example, a new scan of the target subject may be performed after a matter of hours, or the next day, next week, next year or next year. The arrow labelled "time" in Figure 2C represents the possibility of additional scans being added over time (where the different schematized icons labelled 202 in the figure represent different scans taken on different occasions). This provides one or more new images of the target subject, which may be added only after the initial report has been authored and reviewed at least once by the ML model 116. In this case, the method may comprise inputting the new image data from the one or more additional scans into the machine learning model 116, along with the latest version of the report (including any amendments accepted by the user 107). Based on these, the ML model 116 may the output one or more additional suggestions taking into account the new images. These are output to the user 107 through the UI, in order for the user to review and accept or reject them based on his/her judgement, in a similar manner as described earlier.

In embodiments, the training of the machine learning model 116 may also be refined based on the image(s) of the additional scan(s), and optionally any additional amendments which the user 107 accepts, rejects or makes him/herself based on the new scan(s).

It will be appreciated that Figure 2C is just one example workflow. Other methods involving the alignment of text queries with image regions may also be implemented using the ML model disclosed herein.

Figure 3B schematically illustrates an example of the way in which suggested updates may be output to the user 107 through the UI.

An image of the scan 202, or one of the scans, is displayed to the user 107 on a screen of the UI console 106. A version of the report 204 is also displayed to the user on screen, either on the same screen or a different screen of the UI console. The version of the report includes the one or more suggested amendments shown inline in the text, in tracked (marked-up) form. E.g. this may comprise underlining, putting in bold or highlighting any additions; and/or showing any deletions in strike-through. Furthermore, the UI includes a visual effect that links each suggestion to a corresponding area within the image currently on screen (or one of the images), showing which particular part of the image caused the suggestion to be generated. By an area or region *within* the image, or such like, it is meant a particular are smaller than the image itself, i.e. a subregion of the image. There are a number of possible ways in which suggestions in the text and corresponding regions within an image may be linked.

For example, the UI may highlight the suggestion in a certain colour, and highlight the corresponding part of the image in substantially the same colour. If different suggestions are based on different parts of the image, the different suggestions may be highlighted in different colours, and each part of the image may be highlighted in the same colour as the particular suggestion which it generated. In the black & white mock-up of Figure 3B, different types of dotted line are used to schematically represent highlighting in different colours. However another option would indeed be to outline in different line types. A suggested amendment and corresponding image area could be highlighted by the drawing of a box or other shaped line around the text and image area and using a certain colour and/or line type for the box or line, or the highlighting could be by using a certain background colour behind the text and image area, or a tint thereover, or any combination of these effects and/or others.

In the example of Figure 3B, the UI highlights a suggestion by highlighting a whole phrase in which a suggestion is included, and within the highlighted phrase underlines the suggested amendment itself and/or shows it in bold. However this is just one example. Another would be to highlight only the suggested amendment itself.

Another alternative or additional option, as also shown in Figure 3B, is for the UI to mark each suggestion with a particular respective reference sign, such as a letter or numeral, and to mark each corresponding region in the image with the same reference sign. Another example, not shown, would be to display a line connecting the amendment or the phrase containing the amendment to the corresponding region in the image.

If the report is longer than can be shown on screen all at once, the UI may provide the user with the ability to scroll through the report or flip through different pages or sections. Also if the report 204 was based on more than one scan 202, the user 107 may have the option in the UI to flip or scroll through the different scans. If a given scan comprises more than one image (e.g. slice), the user may have the option in the UI to flip or scroll though the different images of the scan. Alternatively or additionally, the UI may automatically switch to one or more images corresponding to whatever section or page of the report the user has selected to view at the moment, or vice versa, automatically switch to the section or page of the report that corresponds to whatever image the user has selected to view at the moment. The UI will show how the suggestions on screen at the time are linked to whatever image is on screen at the time.

The amendment suggested by the ML model 116 could be to suggest some extra detail that the user 107 did not include in the original report. E.g. in phrase 3) in the example of Figure 3B, the user 107 may have omitted to add that the opacities are in the *right* lower lobe, so the ML model 116 suggests to add this in. In some cases the model 116 could even suggest the insertion of a whole phrase that the user did not include, e.g. as in phrase 2) in the example of Figure 3B. Another type of amendment that may be suggested by the ML model is the correction of an error or a change to some terminology in the user's original report, perhaps where the user used language that was potentially misleading, nonstandard or imprecise. E.g. in phrase 1) in Figure 3B, the user may have originally written that the heart size appears "fine", but the ML model 116 may suggests to change this term to "normal" because this is more formal, more standard, and/or more informative or precise. In some embodiments, the UI could also show how any phrases that the ML model 116 agreed with (had no suggestions for) map to a part of the image. E.g. see phrase 4) in Figure 3B. This may give reassurance that the model has still considered this part of the report and shows the part of the image that led the model to agree with the user.

In some embodiments, the UI may enable the user 107 to select a particular suggestion, such as by clicking on, hovering over or touching the suggestion or a phrase containing the suggestion. In response, the UI will emphasize the corresponding region within the image, such as by making the highlighting more intense compared to the other highlighted parts of the image, or causing it to flash. Alternatively or additionally, the UI may enable the user 107 to select a particular highlighted region of the image (or one of the images), such as by clicking on, hovering over or touching the region. In response, the UI will emphasize the corresponding suggestion within the marked-up report, e.g. again by making the highlighting more intense compared to the other highlighted suggestions in the text of the report, or making it flash, etc.

In further alternative or additional embodiments, the machine learning model 116 may also be used to review historical report data. In this case the machine learning model 116 is arranged to search through a plurality of past reports and the associated scans based upon which the reports were originally authored. Based on both the text of the reports and the associated images, the machine learning model 116 may then identify one or more of the historical reports that contains a possible error.

The method may further comprise taking an action based on the indicated link between the text query 204 and the input image 202. The action may comprise applying a treatment to the being, object or substance shown in the input image 202 based on the linkage indicated in the GUI. In the case of a living being this may comprise applying a medical treatment. E.g. this may comprise applying the treatment to the region of the body linked one or more propositions in the text query 204. In the case of an object such as a device, the treatment may comprise repairing, upgrading, refinishing, tuning, debugging the device. E.g. this may comprise repairing, upgrading, refinishing, tuning or debugging a part of the device represented by the region in the image linked to a proposition in the text query. Or the action may comprise clearing the object or substance for release in a quality control process.

Returning to the pre-training stage P10, in embodiments this may be performed over three phases: phase I, phase II and phase III, which are also illustrated in Figure 4 by way of example.

The first phase, phase I, comprises learning a vocabulary of commonly occurring tokens based on a corpus of text data. Preferably this is a domain specific vocabulary specific to the application for which the text model 404 is being trained. E.g. in the medical case this could be a general medical vocabulary, or a medical vocabulary dealing with a specific medical issue. Or in the case of technical diagnostics or analysis, the learned vocabulary may comprise a technical vocabulary relating to a particular field of technology, or a particular type of device or substance. The corpus for the purpose of phase I may be taken from the text database 114. It may comprise some or all of the same corpus 500 that is used for the contrastive learning (phase III). I.e. the vocabulary learned in phase I may be learned from one or both of the first and second text passages 501, 502 of one or more of the text passage combinations based upon which the text encoder 404 is to be pre-trained, as discussed earlier with respect to Figure 5. Alternatively or additionally, the text from which the vocabulary is learned in phase I may comprise some or all of the same textual reports 204 which are used in the joint training P20. As another alternative or additional possibility, the learning of the vocabulary may be performed based on one or more pieces of text that are unique to phase I and not used again in the rest of the method, e.g. medical journals, textbooks or such like.

The learned vocabulary comprises a plurality of commonly occurring tokens found in the relevant corpus. A token may be an individual letter, a part of a word (subword, e.g. "ized" or such like), a whole word, or a short phrase comprising more than one word. Tools for learning a commonly occurring vocabulary in a corpus of text are, in themselves, known in the art. For instance in embodiments the WordPiece tool may be used.

The second phase, phase II, comprises an initial training of the text model 404 based on the learned vocabulary. This may comprise learning a vector representation of each token. The token embeddings may be mapped to the tokens by a look-up table wherein the mapping is a learnable parameter of the text encoder 403. The token embeddings are then mapped to the output text embedding $\tilde{t}$ by the rest of the text encoder 403 (e.g. by one or more neural networks of the encoder).

In embodiments the training in phase II may comprise masked language modelling (MLM). This is performed based on a corpus of MLM training text, which may comprise some or all of the same corpus of text 500 as used in phase I, and/or some or all of the same text as used to perform contrastive learning in phase III, and/or some or all of the reports used in the joint training P20, and/or or some different text which is unique to the MLM training of phase II. In embodiments the MLM training text may comprise some or all of the first and/or second text passages 501, 502, and/or some or all or the vocabulary learning text, and/or different text; or any combination of some or all of the first text passages 501, second text passages 502, vocabulary learning text and additional text.

To perform the MLM training of phase II, the ML engine 118 masks some of the words or tokens of text in the MLM training corpus. E.g. this may be done randomly, or according to a predetermined rule or pattern. Alternatively the MLM training text could be pre-masked in the database 114. Either way, the MLM block 406 comprises a predictor which is arranged to attempt to predict the missing words or tokens that are missing from (i.e. masked in) the masked MLM training text. To do this, a piece of the masked MLM training text is input into the text encoder 403 of the text model 404 in order to generate a token embedding $E_{txt}$ for each token that is present in the piece of MLM training text (i.e. each token that is not masked), based on the token vocabulary that was learned in phase I. The MLM block 406 then attempts to predict the masked tokens or words missing from the piece of MLM training text, and estimates the MLM loss. In phase II, the MLM loss is used to train the predictor (e.g. a small neural network) in the MLM block 406, and also to generate a feedback signal (e.g. a gradient) from the ML block 406 to the text encoder 404 in order to train the text encoder 403. This may be repeated over multiple pieces of masked MLM training text. Subsequently in phase III (discussed shortly),. This feedback signal may comprise a gradient.

The MLM block 406 comprises a MLM language model arranged to make a token prediction for each masked token provided at the input. This is like a multi-choice question where the model is required to choose one token out of N-tokens from a given dictionary. The associated reward/penalty of each prediction is similar to multi-class sample classification, i.e. the MLM block uses a multi-class cross-entropy loss term (negative loglikelihood). The gradient of this loss with respect to each text model parameter is computed to minimise the loss/penalty so that the MLM model can learn to predict the correct token in the next iterations. This helps the MLM model to learn co-occurrence and order of tokens in a given context; in other words, it learns some basic grammar rules based on token statistics seen in training corpus without requiring any human annotations. For instance, articles such as "a" and "the" need to be followed by a noun, or "chest" token is more likely to appear before "scan" token in comparison to "pneumonia" token. Similar analogies can be drawn between MLM-learning and masked-image pre-training, where pixels are masked out from an image and teach the models to guess the missing pixels in the hope to learn the order and co-occurrences of pixels and object-parts.

The third phase, phase III, comprises the contrastive learning described earlier with respective to Figures 2A and 5 - i.e. the training based on the combinations of first and second text passages 501, 502. In this phase, the MLM block 406 may also be used. I.e. the encoder 404 is trained to try to minimize the contrastive loss measured by the loss function 512 and the MLM loss measured by the MLM block 406, weighted according to some weighting between the two loss functions. Hence both in phase II and phase II, the MLM loss measured by the MLM loss function in the MLM block 406 is used to generate a feedback signal to both the text encoder 404 and the predictor in MLM block 406. Thus, it trains these two neural networks in both phase II and phase III. The difference between the two phases is that in phase III we also introduce the RSM loss (512) and down-weight the contribution of the MLM block. The text encoder 404 is responsible for transforming text characters into real-valued embedding representations for each token or word. These features coming out of the text encoder 404 are consumed by the MLM predictor in the MLM block 406 to generate predictions for the masked tokens. This could be described as a composition of two neural networks contributing to the prediction and its resultant error & gradient. Lastly, this penalty signal is then used to train both the text encoder module 404 and the MLM module 406.

Contrasted with phase II, phase III is more related to sentence semantics instead of low-level token statistics. Here the model learns to understand the semantic correspondences between two input sentences or passages. In embodiments, in phase III the MLM block 406 regularises the model to ensure that all token-level information is preserved whilst it's learning sentence semantics in the third phase. For instance, the authors of the present disclosure have observed that the model can discover short-cuts in synonym matching and ignore the relevance of other tokens unless MLM is utilised. The combination of the two objectives aim to capture both the low- and high-level semantic information without requiring labels.

In embodiments, any of the methods disclosed herein may further comprise one or more model evaluation steps T10, T20, T30, T40 as illustrated in Figure 4.

Step T10 comprises a step of local alignment evaluation for evaluating the local alignment performance of the model. This comprises inputting a test image into the image model 401 to generate a respective set of test values of the local image embeddings (i.e. the per spatial unit embeddings), and inputting a textual test proposition to the text model 403 to generate a respective test value of the text embedding. In embodiments these will be the projected versions V, t. The test image is known to contain region that corresponds to the test proposition. The local alignment evaluation then comprises checking whether the ML model 116, under control of the ML engine 118, generates a linkage between the known region in the test image and the text proposition.

Step T20 comprises a step of global alignment evaluation to evaluate the global alignment performance of the ML model 116. This comprises inputting a test image to the image model 401 to generate a respective test value of the global image embedding, and inputting a textual test proposition to the text model 403 to generate a respective test value of the text embedding. In embodiments these will be the projected versions v, t. The test proposition is known to correspond to the test image. The global alignment evaluation then comprises comparing the test value of the image embedding with the test value of the text embedding. The comparison may comprise evaluating a similarity metric such as cosine similarity or mean square error between the vectors of the two embeddings in order to quantify the similarity. The method may comprise determining whether the values are within a threshold similarity of one another according to the similarity metric, and on condition thereof determining that the ML model 116 passes the global alignment evaluation.

Step T30 comprises a step of image model evaluation to evaluate the image model 402. This comprises inputting a plurality of images showing a same entity or type of entity (e.g. same type of being or object), or a same condition (e.g. same disease or illness), in order to generate respective test values of the global image embedding v. The image model evaluation then comprises comparing the test values of the global image embedding. The comparison may comprise evaluating a similarity metric such as cosine similarity or mean square error between the vectors of the two embeddings in order to quantify the similarity. The method may comprise determining whether the values are within a threshold similarity of one another according to the similarity metric, and on condition thereof determining that the image model 402 passes the global alignment evaluation.

Step T40 comprises a step of text model evaluation to evaluate the performance of the text model 403. This comprises inputting a plurality of synonymous text propositions, or at least propositions describing a same matter (e.g. same topic, being or object), into the text model 403 to generate respective test values of the text embedding. In embodiments these will be values of the projected version of the text embedding t. The text model evaluation then comprises comparing the test values of the text embedding. The comparison may comprise evaluating a similarity metric such as cosine similarity or mean square error between the vectors of the two embeddings in order to quantify the similarity. The method may comprise determining whether they are within a threshold similarity of one another according to the similarity metric, and on condition thereof determining that the ML model 116 passes the global alignment evaluation.

and comparing the test values to determine whether they are within a threshold similarity.

EXAMPLE IMPLEMENTATION

The following now describes by way of illustration a particular example implementation of the various techniques disclosed herein.

Advances in deep learning have enabled automated diagnosis systems that operate near or above expert-level performance, paving the way for the use of machine learning systems to improve healthcare workflows, for example by supporting fast triaging and assisting medical professionals to reduce errors and omissions. A hurdle to the widespread development of these systems is a requirement for large amounts of detailed ground-truth clinical annotations for supervised training, which are expensive and time-consuming to obtain. Motivated by this challenge, there has been a rising interest in multi-modal self-supervised learning and cross-modal weak supervision (using partial and imperfect image labels derived from the auxiliary modality), in particular for paired image-text data. Such data is collected daily in routine clinical practice, and common examples are X-ray images or computed tomography scans paired with reports written by qualified medical experts. While many remain private, some paired clinical datasets have been released to the research community including MIMIC-CXR, Open-I, and PadChest.

The following embodiments provide self-supervised vision-language processing (VLP) for paired image and text data in the biomedical domain. The aim is to jointly learn good image and text representations that can be leveraged by downstream applications such as zero- or few-shot image classification, report generation and error detection, and disease localisation. Self-supervised VLP has several advantages over supervised learning, not just because it does not require laborious manual annotations, but also because it does not operate on a fixed number of predetermined conditions or object categories, since the joint latent space is learned from raw text.

However, in contrast to the general domain setting, self-supervised VLP with biomedical data poses additional challenges. Take radiology as an example: publicly available datasets are usually smaller, of the order of a few hundred thousand pairs rather than millions in general-domain vision-language processing. Furthermore, linguistic challenges are different in biomedical settings, including common usage of negations, expressions of uncertainty, long-range dependencies, more frequent spatial relations, the use of domain specific modifiers, as well as scientific terminology rarely found in the general domain. Taking negation as an example, "there is no dog in this picture" would be a highly unusual caption on social media, but "there is no evidence of pneumonia in the left lung" or "there are no new areas of consolidation to suggest the presence of pneumonia" are descriptions commonly found in radiology reports. Moreover, pretrained models including object detectors often used in general domain visual grounding are typically unavailable or underperform in domain-specific applications. Additionally, imbalance in underlying latent entities of interest (e.g., pulmonary findings) can cause larger numbers of false negatives in contrastive learning objectives that sample at random, which can lead models to degrade and memorise irrelevant text and image aspects. For example, radiology images and text reports with normal findings occur much more frequently compared to exams that reveal abnormal conditions such as pneumonia or pneumothorax.

Existing self-supervised VLP work has achieved impressive downstream classification and zero-shot classification performance. However, the authors of the present disclosure have identified that suboptimal text modelling due to insufficient vocabulary adjustment, fine-tuning, and language grounding appears to have gone unnoticed, all of which are shown to degrade the quality of joint latent representations. In particular, a more thorough benchmarking of the text, image, and shared embeddings, across a multitude of downstream benchmarks, reveals that large improvements in performance are possible by taking care to build highly specialised text models and by maintaining their performance during joint training. Free-text image descriptions provide a semantically dense learning signal compared to image-only contrastive methods and supervised classification. Further, extracting shared semantics of images and text pairs is easier for text, as the modality is already discretised. Thus, making the most of text modelling before and during joint training can lead to large improvements in not just the text model, but also of the image model and joint representations.

Embodiments disclosed herein may be used for example to provide the following contributions.

- A new chest X-ray (CXR) domain-specific language model, CXR-BERT1 (Fig. 5). Through an improved vocabulary, a novel pretraining procedure, regularisation, and text augmentation, the model considerably improves radiology natural language inference, radiology masked token prediction, and downstream VLP task performance.
- A simple but effective self-supervised VLP approach for paired biomedical data which may be named herein BioViL (Fig. 4) for use in the radiology setting. Through improvements in text modelling, text model grounding, augmentation, and regularisation, the approach yields improved performance on a wide range of public downstream benchmarks. The disclosed approach may be used for phrase grounding, natural language inference, as well as zero-/few-shot classification and zero-shot segmentation. It achieves improved segmentation performance despite only using a global alignment objective during training.

The ML model 116 of Figure 4 represents a model which in embodiments may be implemented using the presently disclosed BioViL approach, which leverages the disclosed radiology-specific text encoder (CXR-BERT), text augmentation, regularisation, and maintains language model quality via a masked language modelling (MLM) loss. Figure 5 illustrates a text model 403 in which, in embodiments, the text encoder 404 may be implemented as the presently disclosed CXR-BERT text encoder. The text model 403 may employ three phases of pretraining and may use a domain-specific vocabulary, masked language modelling (MLM) and radiology section matching (RSM) losses, regularisation, and text augmentations. Each of the disclosed features is designed to make the most of free-text supervision.

Assume that we are given a set D of pairs of radiology images and reports ($x_{img}$, $x_{txt}$). Let w = ($w_1$, . . . ,$w_T$) denote a vector of T (sub-)word tokens of a text document xtxt (after tokenisation). A BERT encoder Etxt outputs a feature vector for each input token wt as well as a special global [CLS] token used for downstream classification. Let $\tilde{t}$ = $[E_{txt}(w)]_{[CLS]}$ denote the [CLS] token prediction by Etxt based on input w, and t = $P_{txt}(\tilde{t})$ its lower-dimensional projection by a model Ptxt.

The disclosed CXR-BERT encoder (Fig. 5) provides for domain-specific language and model pretraining. It is a specialised CXR language model with an adjusted vocabulary, pretrained in three phases to capture dense semantics in radiology reports. To achieve this specialisation to the CXR report domain despite limited data availability, the disclosed approach includes pretraining on larger data from closely related domains. The phases proceed as follows.

Phase I: First, a custom WordPiece vocabulary of 30k tokens is constructed from PubMed abstracts (15GB), MIMIC-III clinical notes (3.5GB), and MIMICCXR radiology reports (0.1 GB). With this custom vocabulary, the model produces fewer sub-word breakdowns (Table 1).

Table 1: Vocabulary comparison of common radiology terms with ClinicalBERT (Wiki/Book, cased), PubMedBERT (PubMed, uncased), and CXR-BERT (PubMed+MIMIC-III/CXR, uncased). ✓ marks that a word appears in the vocabulary, otherwise its sub-tokens are shown.

| Full word | ClinicalBERT | PubMedBERT | CXR-BERT |
|---|---|---|---|
| pneumonia | ✓ | ✓ | ✓ |
| opacity | op-acity | ✓ | ✓ |
| effusion | e-ff-usion | ✓ | ✓ |
| pneumothorax | p-ne-um-oth-orax | ✓ | ✓ |
| atelectasis | ate-lect-asis | ate-le-ct-asis | ✓ |
| cardiomegaly | card-io-me-gal-y | cardio-me-gal-y | ✓ |
| bibasilar | bi-bas-ila-r | bib-asi-la-r | ✓ |

Phase II: second, a randomly initialised BERT model is pretrained via masked language modelling (MLM) on the PubMed + MIMIC-III + MIMIC-CXR corpora. We largely follow RoBERTa pretraining configurations, i.e. dynamic whole-word masking for MLM and packing of multiple sentences into one input sequence. This phase aims to build an initial domain-specific BERT model in the biomedical and clinical domains.

Phase III: third, pretraining is continued on MIMIC-CXR only to further specialise the CXRBERT encoder to the CXR domain. Here, we also add a novel sequence prediction task to the objective to obtain better sequence representations, as explained below. A raw radiology report xtxt typically consists of several sections, including a 'Findings' section that details clinical observations, and an 'Impression' section summarising the clinical assessment. The sequence prediction objective of phase (III) aims to take advantage of this structure. Specifically, we continually run MLM pretraining on MIMIC-CXR radiology reports and propose to add a radiology section matching (RSM) pretraining task, formulated to match Impression to Findings sections of the same study.

Let θ denote the weights of our language model and m ⊂ {1,..., T} denote mask indices for M masked tokens, randomly sampled for each token vector w at every iteration. Given a batch B of token vectors w = (w1, ...,wT), we write the MLM loss as the cross-entropy for predicting the dynamically masked tokens:

$$\mathcal{L}_{\mathrm{MLM}} = -\frac{1}{|\mathcal{B}|} \sum_{\mathbf{w} \in \mathcal{B}} \log p_\theta(\mathbf{w}_m \mid \mathbf{w}_{\backslash m}) \,.$$

(1)

Further, let $(\tilde{t}_i^F, \tilde{t}_i^I)$ denote a pair of [CLS] tokens corresponding to the Findings and Impression sections of the same $i^{th}$ report, and let $(t_i^F, t_i^I)$ denote the pair projected to a lower dimension via a two-layer perceptron Ptxt. We introduce a contrastive loss on the text modality that favours Impression and Findings text pair from the same report over unmatched ones. Specifically, for a batch of N such pairs, the RSM loss is defined as:

$$\mathcal{L}_{\text{RSM}} = -\frac{1}{N} \sum_{i=1}^{N} \left( \log \frac{\exp(\mathbf{t}_i^F \cdot \mathbf{t}_i^I / \tau_1)}{\sum_{j=1}^{N} \exp(\mathbf{t}_i^F \cdot \mathbf{t}_j^I / \tau_1)} + \log \frac{\exp(\mathbf{t}_i^I \cdot \mathbf{t}_i^F / \tau_1)}{\sum_{j=1}^{N} \exp(\mathbf{t}_i^I \cdot \mathbf{t}_j^F / \tau_1)} \right),$$

(2)

where $\tau 1$ is a scaling parameter to control the margin. The resulting total loss of the specialisation phase-III is $\mathcal{L}_{\text{III}} = \mathcal{L}_{\text{RSM}} + \lambda_{\text{MLM}} \mathcal{L}_{\text{MLM}}$. An additional component for regularising the RSM loss is the use of increased dropout (25%), including on attention. We set $\tau 1 = 0.5$ and $\lambda_{\text{MLM}} = 0.1$, determined by a limited grid-search measuring $\mathcal{L}_{\text{GA}}$ (Eq. (3)) of the joint model on a validation set. We also note that similar losses to the RSM loss, over the same or separate text segments, have been explored successfully for sentence representation learning in other settings. As such, we empirically observed that an objective using masked Findings to Findings matching can achieve similar performance and may be an appropriate replacement in other biomedical settings with differing text structure.

Text Augmentation: as domain-specific datasets are often quite small, effective text augmentation can induce large benefits. In the radiology domain, the sentences of the Findings and Impression sections, which contain the detailed description and summary of the radiological findings, are usually permutation-invariant on the sentence level. We thus find that randomly shuffling sentences within each section is an effective text-augmentation strategy for both pretraining of CXR-BERT as well as during joint model training.

The presently disclosed BioViL approach (Fig. 4) provides for vision-language representation learning, and a simple but effective self-supervised VLP setup for the biomedical domain such as in a chest X-ray (CXR) application setting. BioViL uses a convolutional neural network (CNN) image encoder Eimg, along with the presently disclosed CXR-BERT text encoder Etxt, and projection models $P_{\text{img}}$ and $P_{\text{txt}}$ to learn representations in a joint space. The CNN model allows us to obtain a grid of local image embeddings $\tilde{V} = E_{\text{img}}(x_{\text{img}})$, which is fine-grained enough to be useful for segmentation (e.g. 16x16). Each encoder is followed by a modality-specific two-layer perceptron projection model P, which projects the encoded modality to a joint space of 128 dimensions, e.g. $V = P_{\text{img}}(\tilde{V})$, where the representation is $\ell_2$-normalised. Note that projection should be applied to local embeddings before mean-pooling $v = \text{pool}(P_{\text{img}}(\tilde{V}))$, which gives us the global image embedding v. The text branch uses the Impression section's projected [CLS] token $t^I$ as the text representation in the joint space, as it contains a succinct summary of radiological findings. To align the representations and learn a joint embedding, we propose to use two loss terms. For a batch of size N, a symmetric contrastive loss for global alignment of the image and text projections helps us learn the shared latent semantics:

$$\mathcal{L}_{\text{GA}} = -\frac{1}{N} \sum_{i=1}^{N} \left( \log \frac{\exp(\mathbf{v}_i \cdot \mathbf{t}_i^I / \tau_2)}{\sum_{j=1}^{N} \exp(\mathbf{v}_i \cdot \mathbf{t}_j^I / \tau_2)} + \log \frac{\exp(\mathbf{t}_i^I \cdot \mathbf{v}_i / \tau_2)}{\sum_{j=1}^{N} \exp(\mathbf{t}_i^I \cdot \mathbf{v}_j / \tau_2)} \right).$$

(3)

where $\tau_2$ is a scaling parameter. Further, we maintain the $\mathcal{L}_{\text{MLM}}$ loss (Eq. (1)) during joint training, resulting in the final joint loss $\mathcal{L}_{\text{joint}} = \lambda_{\text{GA}} \mathcal{L}_{\text{GA}} + \mathcal{L}_{\text{MLM}}$. We set $\tau_2 = 0.5$ and $\lambda_{\text{GA}} = 0.5$, determined by a limited grid search measuring $\mathcal{L}_{\text{GA}}$ on a validation set.

Augmentations, Regularisation, and Image Encoder Pretraining: due to the small dataset sizes expected in biomedical applications, we use image and text augmentations to help learn known invariances. We use a ResNet-50 architecture as our image encoder and pretrain the model on MIMIC-CXR images using SimCLR with domain-specific augmentations. For text, we use the same sentence-shuffling augmentation as in pretraining of CXR-BERT. Furthermore, as in phase III of CXRBERT training, we apply higher text encoder dropout (25%) than in standard BERT settings. We find that the combination of all these components, including continuous MLM optimisation, improves downstream performance across the board.

Zero-shot classification: after joint training, we use text prompts to cast the zero-shot classification problem into an image-text similarity task. For C classes, subjectmatter experts design C text prompts representing the target labels c ∈ {1, ...,C}, e.g. for presence or absence of pneumonia. Each class prompt is represented as a vector of tokens $w^c$ and passed to the text encoder and projector of BioViL to obtain $\ell_2$-normalised text features $t^c = P_{txt}(E_{txt}(w^c)) \in \mathbb{R}^{128}$. For each input image $X_{img} \in \mathbb{R}^{H \times W}$, we use the image encoder and projection module to obtain patch embeddings $V = P_{img}(E_{img}(x_{img})) \in \mathbb{R}^{(H/16) \times (W/16) \times 128}$ for segmentation tasks or the pooled embedding $v = pool(V) \in \mathbb{R}^{128}$ for instance-classification. We use dilated convolutions to obtain higher-resolution feature maps. Probabilities for classes/regions can then be computed via a softmax over the cosine similarities between the image (or region) and prompt representations.

*Few-shot tasks with BioViL.* To further assess the representation quality, linear probing is applied to local (V) and global (v) image representations, by learning $\beta \in \mathbb{R}^{128 \times C}$ weights and a bias term. We leverage the pretrained projectors and class text embedding $t^c$ from the zero-shot setting by using them for initialisation, which leads to improved performance and further reduces the need for manual label collection. Specifically, in few-shot classification settings, the weights and bias are initialised with $\beta = [t^1, ...,t^c]$ and zeros, respectively.

## EVALUATING SELF-SUPERVISED BIOMEDICAL VLP

Accurate local alignment between modalities is a beneficial characteristic of successful joint image-text training in healthcare, in particular since image and report samples often contain multiple clinical findings, each of which correspond to distinct image regions. Standard global-alignment approaches may attain high classification accuracy by overfitting to spurious image features for a given finding (e.g. chest tubes in images correlating with mentions of pneumothorax in reports). Image classification, the most frequently evaluated downstream task in related work, requires only scene-level labels, hence a less sophisticated understanding of natural-language image descriptions. Image classification tasks can largely be solved by simply detecting a small set of words and maintaining some understanding of negation, as exemplified by the development of automated, rule-based text-labellers such as CheXpert. Instance-level image-text retrieval tasks address some evaluation limitations, but do not require the level of language reasoning needed to solve local correspondence between phrases and image regions.

With this in mind, the following provides a healthcare equivalent of general domain visual-grounding benchmarks, whilst being mindful of domain-specific radiology language (e.g. paraphrasing and negations). A phrase grounding task would benefit from the ability to parse domain specific location modifiers, the ability to deal with reporting style biases, and understanding of complex negations, all while relating the correct findings to specific image regions. To the best of our knowledge, existing public CXR benchmark datasets to evaluate local aspects of VLP have one or more of the following limitations: bounding boxes without corresponding free text descriptions, a limited number of samples, a limited number of abnormalities, and non-curated phrases impacting evaluation quality.

For evaluation purposes, a data set may be used that contains Chest X-ray bounding box labels paired with radiology text descriptions, annotated and verified by two boardcertified radiologists. Such a dataset may be referred to herein as LACheX - A Chest X-ray Phrase Grounding Benchmark. The phrases in LACheX are not simple short captions, but genuine descriptions of radiological findings from original radiology reports and dictated transcripts. Thus, compared to existing evaluation datasets, the proposed benchmark is a more challenging real-world image-text reasoning task. The LACheX dataset provides 1162 image-sentence pairs of bounding boxes and corresponding phrases, collected across eight different cardiopulmonary radiological findings, with an approximately equal number of pairs for each finding. The dataset comprises instances chosen from the public MIMIC-CXR v2 image-text dataset. To obtain and verify bounding-box annotations, we first obtain MIMIC-CXR samples from a set of studies with pre-existing region proposals, such as ellipses. To link each proposal region with candidate phrases, we sample sentences from the report of each study by extracting the highest matching sentences to the annotated labels using scores of the CheXbert sentence classifier, and also use transcriptions of dictations when available. Next, to better balance findings, we sample additional MIMICCXR studies at random as well as MIMIC-CXR samples used in the ImaGenome dataset, the latter being a dataset of annotations of anatomical regions. These sampled studies do not have pre-existing region proposals.

Radiologists then manually review separate sets of candidates. If a bounding box is not available, the radiologists manually annotate the corresponding region(s) in the image with new bounding boxes. Radiologists reject studies where no correct phrase candidates are available and where existing bounding boxes were placed incorrectly (e.g. covering too large an area). To ensure a high quality, consistent benchmark, the phrase-image samples that do not adhere to our guidelines are filtered out, such as phrases containing multiple abnormalities in distinct lung regions.

## EXPERIMENTS

We have conducted a comprehensive evaluation of the presently-disclosed CXRBERT language model as well as the BioViL self-supervised VLP approach, and compared both to state-of-the art counterparts. We begin by demonstrating CXR-BERT's superior performance and improved vocabulary, including on a radiology-specific NLI benchmark. Next, we assess joint image-and-text understanding of BioViL on our new LACheX benchmark, which evaluates grounding of phrases describing radiological findings to the corresponding image regions. We also investigate zero-shot classification and fine-tuning performance of BioViL on image- and pixel-level prediction tasks via the RSNA pneumonia dataset.

## SET-UP

*Datasets:* we conduct experiments on the MIMIC-CXR v2 chest radiograph dataset, which provides 227,835 imaging studies for 65,379 patients, all collected in routine clinical practice. Each study contains a radiology report and one or more images (377,110 images in total). We only use frontal view scans (AP and PA) and also discard samples without an Impression section. From this data, we establish a training set of 146.7k samples and a set of 22.2k validation samples, ensuring that all samples used for the different downstream evaluations are kept in a held-out test set. We emphasise that no labels are used during pretraining; for early stopping only a loss on validation data is tracked. For evaluation, we use RadNLI to assess the proposed CXR-BERT text model in isolation, the new LACheX assesses joint image-text understanding via phrase grounding, and the RSNA Pneumonia dataset to evaluate zero-shot segmentation, as well as zero-shot and fine-tuned classification performance.

*Image and text pre-processing:* we downsize and centre crop images to a resolution of 512x512 whilst preserving image aspect ratios. We perform image augmentations during training including: random affine transformations, random colour jitter, and horizontal flips (only for image fine-tuning tasks). For text model pre-training we utilise the 'Findings' and 'Impression' sections of reports, while joint training is performed using only the latter. During training, we perform sentence shuffling within sections as text-augmentation. Additionally, we perform limited automatic typo correction.

*Comparison approaches:* we compare the proposed CXR-BERT text model to the other specialised PubMed-BERT and ClinicalBERT models. Note that ClinicalBERT was used in most related studies. We compare BioViL to the closely related, state-of-the-art ConVIRT, LoVT and GLoRIA approaches.

*Metrics:* we report segmentation results via mean intersection over union (mIoU) and contrast-to-noise ratio (CNR), and report the Dice score. We first compute the cosine similarity between a projected phrase embedding t and each element of the local image representation V, resulting in a grid of scores between [-1, 1]. For a given similarity threshold, we compute IoU = |A ∩ B|/|A ∪ B| with A being the true bounding box and B the thresholded region. The mIoU is then defined as an average over the thresholds [0.1, 0.2, 0.3, 0.4, 0.5]. The CNR measures the discrepancy between scores inside and out of the bounding box region, without requiring hard thresholds. This evaluation of local similarities is relevant as some clinical downstream applications may benefit from heatmap visualisations as opposed to discrete segmentations. For CNR, let $A$ and $\bar{A}$ denote the interior and exterior of the bounding box, respectively. We then compute $CNR = |\mu_A - \mu_{\bar{A}}|/\left(\sigma_A^2 - \sigma_{\bar{A}}^2\right)^{1/2}$ , where $\mu x$ and $\sigma_X^2$ are the mean and variance of the similarity values in region X. Finally, the Dice score, defined as 2 |A∩B|/(|A|+|B|), is computed at one fixed threshold.

## TEXT MODEL EVALUATION

*Natural language understanding:* we use the RadNLI benchmark to evaluate how well the proposed CXR-BERT text model captures domain-specific semantics. The dataset contains labelled hypothesis and premise pairs, sourced from MIMIC-CXR radiology reports, with the following label categories: (1) entailment, i.e. the hypothesis can be inferred from the premise; (2) contradiction, i.e. the hypothesis cannot be inferred from the premise; and (3) neutral, i.e. the inference relation is undetermined. RadNLI provides expert-annotated development and test sets (480 examples each), but no official training set. Thus, following, we use MedNLI for training, which has 11k samples sourced from MIMIC-III discharge summaries, with equally distributed NLI labels. We fine-tune the language models up to 20 epochs and use early stopping by monitoring accuracy scores on the RadNLI development set. Table 2 summarises the NLI evaluation, masked token prediction, and subword tokenisation results. Using only MedNLI training samples, our model achieves a good accuracy of 65.21%, and far outperforms fine-tuned ClinicalBERT, PubMedBERT, and the score reported in RadNLI. Another important result is that RadNLI accuracy improves after joint training with images (last row of Table 2).

Table 2: Evaluation of text encoder intrinsic properties and fine-tuning for radiology natural language inference: (1) RadNLI fine-tuning scores (average of 5 runs); (2) Mask prediction accuracy on MIMIC-CXR val. set; (3) Vocabulary comparison, number of tokens vs. original number of words in Findings, increase shown as percentage.

| | RadNLI accuracy (MedNLI transfer) | Mask prediction accuracy | Avg. # of tokens after tokenization | Vocabulary size |
|---|---|---|---|---|
| RadNLI baseline [52] | 53.30 | - | - | - |
| ClinicalBERT | 47.67 | 39.84 | 78.98 (+38.15%) | 28,996 |
| PubMedBEHT | 57.71 | 35.24 | 63.55 (+11.16%) | 28,895 |
| CXR-BERT (after Phase-III) | 60.46 | 77.72 | 58.07 (+1.59%) | 30,522 |
| CXR-BERT (after Phase-III + Joint Training) | 65.21 | 81.58 | 58.07 (+1.59%) | 30,522 |

*Mask prediction accuracy:* while mask prediction accuracy does not always translate to downstream application performance, it is an auxiliary metric that captures important aspects of a language model's grasp of a target domain. We report Top-1 mask prediction accuracy on radiology reports in the MIMICCXR validation set (Table 2), and follow the standard masking configuration (15% masking probability). Despite being trained on closely related data, our CXR-BERT displays a much better mask prediction accuracy compared to ClinicalBERT (trained on MIMIC-III, which includes radiology reports) and Pub-MedBERT (trained on biomedical literature text). This suggests that radiology text significantly differs from other clinical text or biomedical literature text, highlighting the need for specialised text encoder models.

*Ablation:* we also conduct an ablation of the various aspects of CXRBERT, measuring the impact after joint training. Table 3 shows that all components of CXR-BERT contribute to improved downstream and NLI performance, both in terms of alignment between related sentences (entailments) and of discrimination of contradictions. Note the substantial improvement on these scores due to keeping the MLM objective during joint fine tuning.

| Model or pretraining stage | RadNLI | | Grounding | |
|---|---|---|---|---|
| | Syn. sim. | Cont. gap | mIoU | CNR |
| ClinicalBERT | .657 | .609 | .182 | 0.791 |
| Pretrain & Vocab (I-II) | .749 | .646 | .194 | 0.796 |
| + MLM grounding during joint | .871 | .745 | .209 | 0.860 |
| + Use of attention drop-out (III) | .893 | .802 | .217 | 0.945 |
| + RSM Pretrain (III) | .877 | .779 | .220 | 1.012 |
| + Sentence shuffling (CXR-BERT) | .884 | .798 | .220 | 1.031 |

Table 3: CXR-BERT ablation. CNR and mIoU are macro averages of BioViL performance on all categories of LACheX. *Syn. sim.* Denotes the average cosine similarity between RadNLI entailments. *Cont. gap* is the average similarity gap of RadNLI entailment and contradiction pairs. CXR-BERT is the combination of all components below the first row.

LOCAL ALIGNMENT EVALUATION - PHASE GROUNDING

We perform a phrase grounding evaluation of the pretrained BioViL model on the LACheX dataset. For each image-phrase pair, the image is passed to the CNN image encoder and projected to obtain a grid of image representations V in the joint space. Similarly, the phrase is embedded via the text encoder and projected to the joint space to obtain t. Cosine similarity between t and elements of V produces a similarity grid, which is evaluated against the ground-truth bounding boxes. Table 4 shows the superior phrase grounding results achieved by BioViL across radiological findings. We also create BioViL-L by adding a local loss term, which further improves phrase grounding performance for almost all findings. Moreover, the ablation in Table 3 demonstrates that there are clear gains to be had in visual grounding performance by improving the text model.

Table 4: Contrast-to-noise ratio (CNR) obtained on the newly released LACheX dataset, averaged over four runs with different seeds. The results are collected using different text encoder and training objectives (e.g., G&L: Global and local loss).

| Method | Objective | Text encoder | Atelectasis | Cardiomegaly | Consolidation | Lung opacity | Edema | Pneumonia | Pneumothorax | Pl. effusion | Avg. |
|---|---|---|---|---|---|---|---|---|---|---|---|
| Baseline | Global | ClinicalBERT | 0.70 | 0.53 | 1.15 | 0.75 | 0.83 | 0.85 | 0.29 | 1.05 | 0.769 |
| Baseline | Global | PubMedBERT | 0.72 | 0.64 | 1.22 | 0.69 | 0.80 | 0.91 | 0.21 | 0.99 | 0.773 |
| ConVIRT [80] | Global | ClinicalBERT | 0.86 | 0.64 | 1.25 | 0.78 | 0.68 | 1.03 | 0.28 | 1.02 | 0.818 |
| GLoRIA [31] | G&L | ClinicalBERT | 0.08 | 0.53 | 1.38 | 1.05 | 0.66 | 1.18 | 0.47 | 1.20 | 0.930 |
| BioViL | Global | CXR-BERT | 1.02 | 0.63 | 1.42 | 1.05 | 0.93 | 1.27 | 0.48 | 1.40 | 1.027 |
| BioViL-L | G&L | CXR-BERT | 1.17 | 0.95 | 1.45 | 1.19 | 0.96 | 1.19 | 0.74 | 1.50 | 1.142 |

GLOBAL ALIGNMENT EVALUATION - ZERO-SHOT AND FINE-TUNED CLASSIFICATION

To measure the quality of the global alignment, the joint models are also benchmarked on zero-/few-shot binary pneumonia classification problems (image-level) using the external RSNA dataset [64]. Fine-tuning is done via linear probing, i.e. only a last linear layer is trained. The evaluation is conducted on $D_{\text{test}}$ = 9006 images (30% eval. / 70% train.) using the dataset's ground-truth labels. We define two simple text prompts for BioViL, representing presence/absence of pneumonia, namely "Findings suggesting pneumonia" and "No evidence of pneumonia". The image encoders are utilised and fine-tuned as described earlier.

The zero-shot and fine-tuned results in Table 5 show that our focus on better text modelling results in improved joint modelling of shared latent information between text-image pairs. Note that, to achieve superior performance, BioViL does not require extensive human expert text-prompt engineering as for example conducted in GLoRIA, where variations over severity and/or location where created.

Table 5: RSNA Pneumonia zero-shot and fine-tuned classification. We compare to GLoRIA scores reported which outperforms ConVIRT. Training size: GLoRIA (N = 186k, private dataset), BioViL (N = 146.7k of MIMIC-CXR).

| Method | Type | Text model | Loss | % of labels | Acc. | F1 | AUROC |
|---|---|---|---|---|---|---|---|
| SimCLR [7] | Image only | - | Global | 1% | 0.545 | 0.522 | 0.701 |
| | | | | 10% | 0.760 | 0.639 | 0.802 |
| | | | | 100% | 0.788 | 0.675 | 0.849 |
| GLoRIA [31] | Joint | ClinicalBERT | Global & local | Zero-shot | 0.70 | 0.58 | - |
| | | | | 1% | 0.72 | 0.63 | 0.861 |
| | | | | 10% | 0.78 | 0.63 | 0.880 |
| | | | | 100% | 0.79 | 0.65 | 0.886 |
| Baseline | Joint | ClinicalBERT | Global | Zero-shot | 0.719 | 0.614 | 0.812 |
| BioViL | Joint | CXR-BERT | Global | Zero-shot | 0.732 | 0.665 | 0.831 |
| | | | | 1% | 0.805 | 0.723 | 0.881 |
| | | | | 10% | 0.812 | 0.727 | 0.884 |
| | | | | 100% | 0.822 | 0.733 | 0.891 |

LOCAL ALIGNMENT EVALUATION - SEMANTIC SEGMENTATION

We evaluate models on an RSNA pneumonia segmentation task, using grid-level image representations in the joint latent space. We use the same text prompts as in the previous section for all models, and evaluate against ground-truth bounding boxes of the RSNA pneumonia dataset ( $|\mathcal{D}_{\text{train}}| = 6634$ and $|\mathcal{D}_{\text{test}}| = 2907$ ). Table 6 shows that BioViL significantly reduces the need for dense annotations as compared to similar multi-modal and image-only pretraining approaches, outperforming them when using the same number of labelled data points. Note that our proposed modelling framework BioViL (Fig .4), uses neither a local loss term, nor a separate object detection or segmentation network. Further, while Table 6 shows results using two simple queries, we find that BioViL continues to outperform related work even when more prompts are used for all models. Dice and IoU are computed using the same threshold of 0.6 on predictions scaled between [0, 1].

Table 6: RSNA pneumonia segmentation. Related work is reproduced in the same experimental setup except for LoVT. Zero-shot and linear probing results demonstrate the effectiveness of learning and pretraining with free-text data.

| Method | % of Labels | Supervision | IoU | Dice | CNR |
|---|---|---|---|---|---|
| LoVT (54) | 100% | Lin. prob. | - | 0.518 | - |
| ConVIRT (80) | - | Zero-shot | 0.228 | 0.348 | 0.849 |
| GLoRIA [31] | - | Zero-shot | 0.245 | 0.366 | 1.052 |
| BioViL | - | Zero-shot | 0.355 | 0.496 | 1.477 |
| SimCLR [7] | 5 % | Lin. prob. | 0.382 | 0.525 | 1.722 |
| SimCLR [7] | 100% | Lin. prob. | 0.427 | 0.570 | 1.922 |
| BioViL | 5% | Lin. prob. | 0.446 | 0.592 | 2.07 7 |

(continued)

| Method | % of Labels | Supervision | IoU | Dice | CNR |
|--------|-------------|-------------|-----|------|-----|
| BioViL | 100% | Lin. prob. | 0.469 | 0.614 | 2.178 |

FURTHER REMARKS

It will be appreciated that the above embodiments have been disclosed by way of example only.

More generally, according to one aspect disclosed herein, there is provided computer-implemented method comprising: accessing a machine learning model comprising an image model and a text model; accessing a plurality of text passage combinations, each text passage combination comprising a respective first text passage and a respective second text passage describing a same matter as the respective first text passage but being differently worded than the respective first text passage; performing first training of the text model, wherein the first training comprises inputting each first text passage into the text model and thereby generating a respective value of a first text embedding, inputting each second text passage into the text model and thereby generating a corresponding value of a second text embedding, and training the text model by minimizing a measure of statistical difference between the value of the first text embedding and the corresponding value of the second text embedding over the plurality of text passage combinations; accessing a plurality of image-text combinations, each comprising a respective image and a respective textual report describing the respective image; and after the first training, performing joint training of the image model and text model, the joint training comprising inputting each image into the image model and thereby generating a respective value of an image embedding, inputting each textual report into the text model and thereby generating a corresponding value of a third text embedding, and training the image model and text model by minimizing a measure of statistical difference between the value of the image embedding and the corresponding value of the third text embedding over the plurality of image-text combinations.

In embodiments, the first training may additionally comprise: learning a vocabulary of commonly occurring tokens from a corpus of vocabulary training text, each token comprising a letter, subword, word or phrase; and training the text model based on the learned vocabulary.

In embodiments, said training of the text model based on the learned vocabulary may comprise language masking modelling, MLM, comprising: masking one or more parts of at least one piece of MLM training text and thereby producing masked MLM training text; inputting the masked MLM text to the text model and thereby generating, based on the tokens from the learned vocabulary, a token embedding for each of a plurality of instances of the tokens found in the masked MLM text; and predicting at least one of the one or more masked parts of the MLM training text based on at least some of the token embeddings found in the masked MLM text, and based thereon providing a feedback signal to the text encoder to train the text encoder.

In embodiments, said first training may be performed over a first phase, a second phase following the second phase, and a third phase following the second phase; wherein: the first phase comprises the learning of the vocabulary, the second phase comprises the MLM, and the third phase comprises said inputting of the first and second text passages and said training of the text model to minimize the statistical distance between the value of the first and corresponding second text embedding.

In embodiments, the method may further comprise either or both of:

- shuffling sentences in at least one of the first text passages or second text passages prior to the inputting into the text model in the first training, or
- shuffling sentences in at least one of the textual reports prior to the inputting into the text model in the joint training.

In embodiments, each image may comprise an image of the matter described by a respective one of the first text passage and/or a respective one of the second text passages, and each textual report may comprise the respective first and/or second passage.

In embodiments, for at least some of the first text passages, any one of:

- the first text passage may be a findings section of a respective document and the respective second passage may be an impressions section of the respective document,
- the first passage may be an abstract of a document and the respective second passage may be at least part of a body of the respective document,
- the first passage may be a translation of the second text passage or vice versa or both may be translations of a same source passage,
- the first text passage and respective second text passage may be descriptions of the same matter by different authors, or

- at least one of the first passage and respective second passages may comprise automatically generated text augmentations.

In embodiments, each image may be divided into a plurality of spatial units; wherein for each image input to the image model, the image model may be configured to generate a local embedding per spatial unit of the input image. In this case the generating of the respective image embedding for each image may comprise combining the local embeddings of the respective image into the respective image embedding.

In embodiments, the text model may comprise a text encoder and a text projector, and the image model may comprise an image encoder and an image projector. In this case, for each image-text combination: the image encoder may be arranged to generate an unprojected embedding per spatial unit of the respective image, and the image projector may be arranged to project each of the unprojected embeddings into the local embedding per spatial unit, to be combined into the respective image embedding; and the text encoder may be arranged to generate an unprojected embedding of the respective textual report, and the text projector may be arranged to project the unprojected embedding of the respective textual report into the respective third text embedding, such that the third text embedding and respective image embedding.

In embodiments, each of the spatial units comprises a respective plurality of pixels of a respective sub-area of the image, and the generating of each local embedding comprises combining the respective plurality of pixels using a dilated convolution operation.

In embodiments the method may comprise, after the first training and joint training: receiving a text query and a target image; inputting the target image into the image model and thereby generating a local embedding per spatial unit of the target image, and inputting the text query into the text model to generate a corresponding text embedding; comparing the text embedding of the text query with each of a plurality of the local embeddings of the target image and thereby linking the text query to a region of the target image; and outputting graphical data for rendering, on a display, graphical information indicating the linking between the region of the target image and the text query.

In an example use case, the matter described by the first and second text passages in at least some of the text passage combinations in the first training may comprises a description of a medical condition affecting a body part of a species of living being, each of the images in the joint training may comprise an image of a respective instance of the body part in a respective member of said species, and each respective textual report may comprise a medical report describing an occurrence or absence of the medical condition in the respective instance of the body part. In this case the target image may comprise an image of an instance of the body part in a subject being a member of said species, and the text query may comprise a proposition about possible presence or absence of the medical condition in the target instance of the species.

In another example use case, the matter described by the first and second text passages in at least some of the text passage combinations in the first training may comprise a physical condition affecting a type of physical object (e.g. device) or substance, each of the images in the joint training may comprise an image of at least part of an instance of said type of physical object or substance, and each of the respective textual reports may describe an occurrence or absence of the physical condition in the respective instance of the object or substance. In this case the target image may comprise an image of at least part of a specimen instance of said type of physical object or substance, and the text query may comprise a proposition about possible presence or absence of the physical condition in the specimen instance.

In another example use case, the matter described by the first and second text passages in at least some of the text passage combinations in the first training may comprise presence or absence of a species of being or type of object or one or more physical features of the species of living being or type of object, each of the images in the joint training may comprise an image of at least part of a member of said species of living being or of an instance of said type object, and each of the respective textual reports may describe a presence or absence of at least part of the respective member of the species of living being or instance of the type of object. In this case the text query may comprise a proposition about possible presence or absence of at least part of a member of the species of living being or of an instance of the type of object.

In embodiments, the method may comprise, after the first training and the joint training, performing a local alignment evaluation of the ML model by inputting a test image to the image model to generate a test value for each of a plurality of local embeddings each encoding a different spatial unit of the test image, and inputting a test proposition to the text model to generate a respective test value of the text embedding, in order to use the ML model to align the text proposition with an automatically detected region in the test image based on a comparison between the test value of the text embedding and the test values of the local embeddings, wherein the test proposition is known to relate to a known region in the test image, and the local alignment evaluation comprises determining whether the automatically-detected region corresponds to the known region.

In embodiments, the method may comprise, after the first training and the joint training, performing a global alignment evaluation of the ML model by inputting a test image to the image model to generate a respective test value of the image embedding, inputting a test proposition to the text model to generate a respective text value of the text embedding, and comparing the test value of the image embedding with the test value of the text embedding.

In embodiments, the method may comprise, after the first training and the joint training, evaluating the image model by inputting a plurality of test images showing a same entity or condition into the image model to generate respective test values of the image embedding, and comparing the test values of the image embedding.

In embodiments, the method may comprise, after the first training and the joint training, evaluating the text model by inputting a plurality of synonymous test propositions, or propositions describing a same matter, into the text model to generate respective test values of the text embedding, and comparing the test values of the text embedding.

Other variants or use cases may become apparent to a person skilled in the art once given the disclosure herein. The scope of the present disclosure is not limited by the above-described embodiments, but only by the accompanying claims.

**Claims**

1. A computer-implemented method comprising:

   accessing a machine learning model comprising an image model and a text model;
   accessing a plurality of text passage combinations, each text passage combination comprising a respective first text passage and a respective second text passage describing a same matter as the respective first text passage but being differently worded than the respective first text passage;
   performing first training of the text model, wherein the first training comprises:

   - inputting each first text passage into the text model and thereby generating a respective value of a first text embedding,
   - inputting each second text passage into the text model and thereby generating a corresponding value of a second text embedding, and
   - training the text model by minimizing a measure of statistical difference between the value of the first text embedding and the corresponding value of the second text embedding over the plurality of text passage combinations;

   accessing a plurality of image-text combinations, each comprising a respective image and a respective textual report describing the respective image; and
   after the first training, performing joint training of the image model and text model, the joint training comprising:

   - inputting each image into the image model and thereby generating a respective value of an image embedding,
   - inputting each textual report into the text model and thereby generating a corresponding value of a third text embedding, and
   - training the image model and text model by minimizing a measure of statistical difference between the value of the image embedding and the corresponding value of the third text embedding over the plurality of image-text combinations.

2. The method of claim 1, wherein the first training additionally comprises:

   learning a vocabulary of commonly occurring tokens from a corpus of vocabulary training text, each token comprising a letter, subword, word or phrase; and
   training the text model based on the learned vocabulary.

3. The method of claim 2, wherein said training of the text model based on the learned vocabulary comprises language masking modelling, MLM, comprising:

   - masking one or more parts of at least one piece of MLM training text and thereby producing masked MLM training text,
   - inputting the masked MLM text to the text model and thereby generating, based on the tokens from the learned vocabulary, a token embedding for each of a plurality of instances of the tokens found in the masked MLM text, and
   - predicting at least one of the one or more masked parts of the MLM training text based on at least some of the token embeddings found in the masked MLM text, and based thereon providing a feedback signal to the text encoder to train the text encoder.

4. The method of claim 3, wherein said first training is performed over a first phase, a second phase following the second phase, and a third phase following the second phase; wherein:

the first phase comprises the learning of the vocabulary,
the second phase comprises the MLM, and
the third phase comprises said inputting of the first and second text passages and said training of the text model to minimize the statistical distance between the value of the first and corresponding second text embedding.

5. The method of any preceding claim, comprising either or both of:

- shuffling sentences in at least one of the first text passages or second text passages prior to the inputting into the text model in the first training, or
- shuffling sentences in at least one of the textual reports prior to the inputting into the text model in the joint training.

6. The method of any preceding claim, wherein:

each image comprises an image of the matter described by a respective one of the first text passage and/or a respective one of the second text passages, and
each textual report comprises the respective first and/or second passage.

7. The method of any preceding claim, wherein for at least some of the first text passages, any one of:

- the first text passage is a findings section of a respective document and the respective second passage is an impressions section of the respective document,
- the first passage is an abstract of a document and the respective second passage is at least part of a body of the respective document,
- the first passage is a translation of the second text passage or vice versa or both are translations of a same source passage,
- the first text passage and respective second text passage are descriptions of the same matter by different authors, or
- at least one of the first passage and respective second passages comprises automatically generated text augmentations.

8. The method of any preceding claim, wherein:

each image is divided into a plurality of spatial units;
for each image input to the image model, the image model is configured to generate a local embedding per spatial unit of the input image; and
the generating of the respective image embedding for each image comprises combining the local embeddings of the respective image into the respective image embedding.

9. The method of claim 8, wherein the text model comprises a text encoder and a text projector, and the image model comprises an image encoder and an image projector; wherein for each image-text combination:

the image encoder is arranged to generate an unprojected embedding per spatial unit of the respective image, and the image projector is arranged to project each of the unprojected embeddings into the local embedding per spatial unit, to be combined into the respective image embedding; and
the text encoder is arranged to generate an unprojected embedding of the respective textual report, and the text projector is arranged to project the unprojected embedding of the respective textual report into the respective third text embedding, such that the third text embedding and respective image embedding.

10. The method of claim 8 or 9, wherein each of the spatial units comprises a respective plurality of pixels of a respective sub-area of the image, and the generating of each local embedding comprises combining the respective plurality of pixels using a dilated convolution operation.

11. The method of any of claims 8 to 10 comprising, after the first training and joint training:

receiving a text query and a target image;
inputting the target image into the image model and thereby generating a local embedding per spatial unit of the target image, and inputting the text query into the text model to generate a corresponding text embedding;
comparing the text embedding of the text query with each of a plurality of the local embeddings of the target image and thereby linking the text query to a region of the target image; and
outputting graphical data for rendering, on a display, graphical information indicating the linking between the region of the target image and the text query.

**12.** The method of claim 11, wherein any one of:

- the matter described by the first and second text passages in at least some of the text passage combinations in the first training comprises a description of a medical condition affecting a body part of a species of living being, each of the images in the joint training comprise an image of a respective instance of the body part in a respective member of said species, each respective textual report comprises a medical report describing an occurrence or absence of the medical condition in the respective instance of the body part, the target image comprises an image of an instance of the body part in a subject being a member of said species, and the text query comprises a proposition about possible presence or absence of the medical condition in the target instance of the species; or
- the matter described by the first and second text passages in at least some of the text passage combinations in the first training comprises a physical condition affecting a type of physical object or substance, each of the images in the joint training comprises an image of at least part of an instance of said type of physical object or substance, each of the respective textual reports describes an occurrence or absence of the physical condition in the respective instance of the object or substance, the target image comprises an image of at least part of a specimen instance of said type of physical object or substance, and the text query comprises a proposition about possible presence or absence of the physical condition in the specimen instance; or
- the matter described by the first and second text passages in at least some of the text passage combinations in the first training comprises presence or absence of a species of being or type of object or one or more physical features of the species of living being or type of object, each of the images in the joint training comprises an image of at least part of a member of said species of living being or of an instance of said type object, each of the respective textual reports describes a presence or absence of at least part of the respective member of the species of living being or instance of the type of object, and the text query comprises a proposition about possible presence or absence of at least part of a member of the species of living being or of an instance of the type of object.

**13.** The method of any preceding claim comprising, after the first training and the joint training, performing any one or more of:

- performing a local alignment evaluation of the ML model by inputting a test image to the image model to generate a test value for each of a plurality of local embeddings each encoding a different spatial unit of the test image, and inputting a test proposition to the text model to generate a respective test value of the text embedding, in order to use the ML model to align the text proposition with an automatically detected region in the test image based on a comparison between the test value of the text embedding and the test values of the local embeddings, wherein the test proposition is known to relate to a known region in the test image, and the local alignment evaluation comprises determining whether the automatically-detected region corresponds to the known region; or
- performing a global alignment evaluation of the ML model by inputting a test image to the image model to generate a respective test value of the image embedding, inputting a test proposition to the text model to generate a respective test value of the text embedding, and comparing the test value of the image embedding with the test value of the text embedding; or
- evaluating the image model by inputting a plurality of test images showing a same entity or condition into the image model to generate respective test values of the image embedding, and comparing the test values of the image embedding; or
- evaluating the text model by inputting a plurality of synonymous test propositions, or propositions describing a same matter, into the text model to generate respective test values of the text embedding, and comparing the test values of the text embedding.

**14.** A computer system comprising:

processing apparatus comprising one or more processors, and
memory comprising one or more memory units;
wherein the memory stores code arranged to run on the processing apparatus, the code being configured so as when run to perform the method of any of claims 1 to 13.

15. A computer program comprising code embodied on a computer storage medium or media, the code being configured so as when run on one or more processors to perform the method of any of claims 1 to 13.

# Figure 1

# Figure 2A

| Pre-training of text model | Pre-training of text model — P10 |
| P15 | |

Joint training of image model and text model — P20

# Figure 2B

Receive input image — R10

Receive text query — R20

Generate local image embeddings — R30

Generate text embedding — R40

Align — R50

Output image with text query matched to region in image — R60

# Figure 2C

Figure 3A

Figure 3B

Figure 4

# Figure 5

EP 4 266 195 A1

**500** Pretraining Corpora

- MIMIC-CXR Reports
- MIMIC-III Clinical Notes

$\mathcal{D}$

**Phase-I** → Bio+Clinical Vocabulary

**Phase-II** → MLM Pretraining with All Corpora

**Phase-III - Specialize Model for Chest X-Ray Reports**

Radiology Report $x_{txt} \in \mathcal{D}$

**501 Findings Section**
- "Lung volumes [MASK] low."
- "[MASK] cardiac silhouette is unremarkable."
- "The pulmonary vasculature [MASK] normal."
- "No pleural effusion or pneumothorax."
- "No focal consolidation is identified."

**502 Impression Section**
- "No [MASK] intrathoracic abnormality."
- "Specifically, no evidence [MASK] edema"

**422** Shuffle sentences $w^F$

**422** Shuffle sentences $w^I$

**404** CXR-BERT Text Encoder

[CLS] $\tilde{t}^F$

[CLS] $\tilde{t}^I$

$E_{txt}(w^F)$

$E_{txt}(w^I)$

**410** Text Proj.

$t^F$

$t^I$

**403**

**406** MLM loss

**406** MLM loss

**512** RSM loss

**204**

Europäisches
Patentamt

European
Patent Office

Office européen
des brevets

## EUROPEAN SEARCH REPORT

Application Number

EP 22 16 8875

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Müller Philip ET AL: "JOINT LEARNING OF LOCALIZED REPRESENTATIONS FROM MEDICAL IMAGES AND REPORTS", , 6 December 2021 (2021-12-06), XP055967126, Retrieved from the Internet: URL:https://arxiv.org/pdf/2112.02889v1.pdf [retrieved on 2022-10-03] * the whole document * | 1-15 | INV. G06F16/45 G06F16/55 G16H30/00 G16H50/20 G06N3/04 G06N3/08 |
| X | & Jacob Devlin ET AL: "BERT: Pre-training of Deep Bidirectional Transformers for Language Understanding", , 24 May 2019 (2019-05-24), XP055723406, Retrieved from the Internet: URL:https://arxiv.org/pdf/1810.04805v2.pdf [retrieved on 2020-08-18] * the whole document * | 1-15 | |
| X | & EMILY ALSENTZER ET AL: "Publicly Available Clinical BERT Embeddings", ARXIV.ORG, CORNELL UNIVERSITY LIBRARY, 201 OLIN LIBRARY CORNELL UNIVERSITY ITHACA, NY 14853, 6 April 2019 (2019-04-06), XP081370463, * the whole document * ----- | 1-15 | TECHNICAL FIELDS SEARCHED (IPC) G16H G06F G06N |

The present search report has been drawn up for all claims

| Place of search | Date of completion of the search | Examiner |
|---|---|---|
| The Hague | 3 October 2022 | Ntarlagiannis, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)